# EUROPEAN PATENT APPLICATION

(11) **EP 2 722 670 A1**
(43) Date of publication of application: **23.04.2014**
(21) Application number: 12800892.7
(22) Date of filing: 15.06.2012
(51) Int. Cl.: G01N 33/53, G01N 33/574

(54) **METHOD FOR ANALYZING MUCIN 1 USING PROBE CAPABLE OF BINDING TO 3´-SULFONATED CORE 1 CARBOHYDRATE CHAIN, AND METHOD FOR DETECTING OR MONITORING BREAST CANCER**

(30) Priority: 16.06.2011 JP 2011134350
(71) Applicant: Tokyo Institute of Technology, Tokyo 152-8550 (JP); Yamaguchi University, Yamaguchi 753-8511 (JP)
(72) Inventor: YAMASHITA, Katsuko, Tokyo 152-8550 (JP); IDEO, Hiroko, Tokyo 152-8550 (JP); HINODA, Yuji, Ube-shi Yamaguchi 755-8505 (JP)
(74) Representative: Cabinet Plasseraud
(86) International application number: PCT/JP2012/065360
(87) International publication number: WO 2012/173228

(57) **Abstract**

The purpose of the present invention is to provide a method for detecting even mucin 1 derived from breast cancer, which cannot be detected by a CA15-3 measurement method.

The purpose can be achieved by a method for analyzing the mucin 1 having a Sulfo-3Galβ1-3GalNAc-R carbohydrate chain, including: a step of bringing a 3' sulfonated core 1 carbohydrate chain binding probe capable of binding to the Sulfo-3Galβ1-3GalNAc-R carbohydrate chain into contact with a sample to be tested; a step of bringing a 3' sulfonated core 1 carbohydrate chain mucin 1 peptide binding probe capable of binding to the mucin 1 having a Sulfo-3Galβ1-3GalNAc-R carbohydrate chain or a mucin 1 binding probe capable of binding to a mucin 1 into contact with the sample to be tested; and a step of detecting a conjugate of the mucin 1 having a Sulfo-3Galβ1-3GalNAc-R carbohydrate chain and probe.

SEQUENCE LISTING

<110>  TOKYO INSTITUTE OF TECHNOLOGY
       YAMAGUCHI UNIVERSITY

<120>  Method for analyzing mucin 1 having sulfated sugar chain and method
for detecting or monitoring breast cancer

<130>  TIT-905

<150>  JP 2011-134350
<151>  2011-06-16

<160>  18

<170>  PatentIn version 3.1

<210>  1
<211>  972
<212>  DNA
<213>  Homo sapiens

<400>  1
atggcctatg tccccgcacc gggctaccag cccacctaca acccgacgct gccttactac      60

cagcccatcc cgggcgggct caacgtggga atgtctgttt acatccaagg agtggccagc     120

gagcacatga agcggttctt cgtgaacttt gtggttgggc aggatccggg ctcagacgtc     180

gccttccact tcaatccgcg gtttgacggc tgggacaagg tggtcttcaa cacgttgcag     240

ggcgggaagt ggggcagcga ggagaggaag aggagcatgc ccttcaaaaa gggtgccgcc     300

tttgagctgg tcttcatagt cctggctgag cactacaagg tggtggtaaa tggaaatccc     360

ttctatgagt acgggcaccg gcttccccta cagatggtca cccacctgca agtggatggg     420

gatctgcaac ttcaatcaat caacttcatc ggaggccagc ccctccggcc ccagggaccc     480

ccgatgatgc caccttaccc tggtcccgga cattgccatc aacagctgaa cagcctgccc     540

accatggaag gacccccaac cttcaacccg cctgtgccat atttcgggag gctgcaagga     600

gggctcacag ctcgaagaac catcatcatc aagggctatg tgcctcccac aggcaagagc     660

tttgctatca acttcaaggt gggctcctca ggggacatag ctctgcacat taatccccgc     720

atgggcaacg gtaccgtggt ccggaacagc cttctgaatg gctcgtgggg atccgaggag     780

aagaagatca cccacaaccc atttggtccc ggacagttct ttgatctgtc cattcgctgt     840

ggcttggatc gcttcaaggt ttacgccaat ggccagcacc tctttgactt tgcccatcgc     900

ctctcggcct tccagagggt ggacacattg gaaatccagg gtgatgtcac cttgtcctat     960

gtccagatct aa                                                         972


<210>  2
<211>  323
<212>  PRT
<213>  Homo sapiens

<400>  2

Met Ala Tyr Val Pro Ala Pro Gly Tyr Gln Pro Thr Tyr Asn Pro Thr
1               5                   10                  15

Leu Pro Tyr Tyr Gln Pro Ile Pro Gly Gly Leu Asn Val Gly Met Ser
            20                  25                  30

Val Tyr Ile Gln Gly Val Ala Ser Glu His Met Lys Arg Phe Phe Val
        35                  40                  45

Asn Phe Val Val Gly Gln Asp Pro Gly Ser Asp Val Ala Phe His Phe
    50                  55                  60

Asn Pro Arg Phe Asp Gly Trp Asp Lys Val Val Phe Asn Thr Leu Gln
65                  70                  75                  80

Gly Gly Lys Trp Gly Ser Glu Glu Arg Lys Arg Ser Met Pro Phe Lys
                85                  90                  95

Lys Gly Ala Ala Phe Glu Leu Val Phe Ile Val Leu Ala Glu His Tyr
            100                 105                 110

Lys Val Val Val Asn Gly Asn Pro Phe Tyr Glu Tyr Gly His Arg Leu
        115                 120                 125

Pro Leu Gln Met Val Thr His Leu Gln Val Asp Gly Asp Leu Gln Leu
    130                 135                 140

Gln Ser Ile Asn Phe Ile Gly Gly Gln Pro Leu Arg Pro Gln Gly Pro
145                 150                 155                 160

Pro Met Met Pro Pro Tyr Pro Gly Pro Gly His Cys His Gln Gln Leu
                165                 170                 175

Asn Ser Leu Pro Thr Met Glu Gly Pro Pro Thr Phe Asn Pro Pro Val
            180                 185                 190

Pro Tyr Phe Gly Arg Leu Gln Gly Gly Leu Thr Ala Arg Arg Thr Ile
        195                 200                 205

Ile Ile Lys Gly Tyr Val Pro Pro Thr Gly Lys Ser Phe Ala Ile Asn
    210                 215                 220

Phe Lys Val Gly Ser Ser Gly Asp Ile Ala Leu His Ile Asn Pro Arg
225                 230                 235                 240

Met Gly Asn Gly Thr Val Val Arg Asn Ser Leu Leu Asn Gly Ser Trp

245                 250                 255

Gly Ser Glu Glu Lys Lys Ile Thr His Asn Pro Phe Gly Pro Gly Gln
            260                 265                 270

Phe Phe Asp Leu Ser Ile Arg Cys Gly Leu Asp Arg Phe Lys Val Tyr
        275                 280                 285

Ala Asn Gly Gln His Leu Phe Asp Phe Ala His Arg Leu Ser Ala Phe
    290                 295                 300

Gln Arg Val Asp Thr Leu Glu Ile Gln Gly Asp Val Thr Leu Ser Tyr
305                 310                 315                 320

Val Gln Ile

<210>  3
<211>  972
<212>  DNA
<213>  Sus scrofa

<400>  3
atggccttcg tccctgcacc aggctaccag cccacctaca atcccacgct gccctactac      60

aagcccatcc caggcggtct ccgggtcgga atgtccgttt acatccaagg agtggccaac     120

gagcacatga agaggttctt cgtgaacttc gtggtggggc agggcccggg ggcggatgtc     180

gccttccact tcaatcctcg cttcgatggc tgggacaagg tggtcttcaa ctcgcagcag     240

gacggcaagt ggggcaacga ggagaagaag aggagcatgc ccttccgcaa ggcgcccgcc     300

ttcgagctgg tcatcatggt cctgcccgag cactacaagg tggtggtaaa cggtgatccc     360

ttctatgagt ttgggcaccg gatcccagtc cagttggtca cccacctgca agtggatggg     420

gacctgacgc ttcaatcaat caacttcatc ggaggccagc ccgcccccag cccgggaccc     480

atgcctaatc cggggtaccc aggtcctgga aagcacaacc aacagccgtg taacctgcca     540

tgcatggagg gagccccaac cttcaacccg cctgtgccat ataagacgag actgcaaggg     600

ggccttgtgg cccgaagaac catcgtgatc aagggctatg tgcccccctc gggcaagagc      660

cttgtcatca acttcaaggt gggctcctca ggggacgtgg ctttgcacat caacccccgc     720

ctgaccgagg gcatcgtggt tcggaacagc tatctgaatg gcaagtgggg agccgaggag     780

aggaagagct ccttcaaccc gtttgctccc ggacagtact tcgatctgtc cattcgctgt     840

ggcttggatc gcttcaaggt ttacgccaat ggccagcacc tcttcgactt ctcccatcgc     900

ctctcgaact tccaaggggt ggacacactg gagatccagg gcgatgtcac cttgtcctac     960

gtccagatct ga                                                         972

<210>  4
<211>  323
<212>  PRT
<213>  Sus scrofa

<400>  4

Met Ala Phe Val Pro Ala Pro Gly Tyr Gln Pro Thr Tyr Asn Pro Thr
1               5                   10                  15

Leu Pro Tyr Tyr Lys Pro Ile Pro Gly Gly Leu Arg Val Gly Met Ser
            20                  25                  30

Val Tyr Ile Gln Gly Val Ala Asn Glu His Met Lys Arg Phe Phe Val
        35                  40                  45

Asn Phe Val Val Gly Gln Gly Pro Gly Ala Asp Val Ala Phe His Phe
    50                  55                  60

Asn Pro Arg Phe Asp Gly Trp Asp Lys Val Val Phe Asn Ser Gln Gln
65                  70                  75                  80

Asp Gly Lys Trp Gly Asn Glu Glu Lys Lys Arg Ser Met Pro Phe Arg
                85                  90                  95

Lys Ala Pro Ala Phe Glu Leu Val Ile Met Val Leu Pro Glu His Tyr
            100                 105                 110

Lys Val Val Val Asn Gly Asp Pro Phe Tyr Glu Phe Gly His Arg Ile
        115                 120                 125

Pro Val Gln Leu Val Thr His Leu Gln Val Asp Gly Asp Leu Thr Leu
    130                 135                 140

Gln Ser Ile Asn Phe Ile Gly Gly Gln Pro Ala Pro Ser Pro Gly Pro
145                 150                 155                 160

Met Pro Asn Pro Gly Tyr Pro Gly Pro Gly Lys His Asn Gln Gln Pro
                165                 170                 175

Cys Asn Leu Pro Cys Met Glu Gly Ala Pro Thr Phe Asn Pro Pro Val
            180                 185                 190

Pro Tyr Lys Thr Arg Leu Gln Gly Gly Leu Val Ala Arg Arg Thr Ile
        195                 200                 205

Val Ile Lys Gly Tyr Val Pro Pro Ser Gly Lys Ser Leu Val Ile Asn
    210                 215                 220

Phe Lys Val Gly Ser Ser Gly Asp Val Ala Leu His Ile Asn Pro Arg
225                 230                 235                 240

Leu Thr Glu Gly Ile Val Val Arg Asn Ser Tyr Leu Asn Gly Lys Trp
                245                 250                 255

Gly Ala Glu Glu Arg Lys Ser Ser Phe Asn Pro Phe Ala Pro Gly Gln
            260                 265                 270

Tyr Phe Asp Leu Ser Ile Arg Cys Gly Leu Asp Arg Phe Lys Val Tyr
        275                 280                 285

Ala Asn Gly Gln His Leu Phe Asp Phe Ser His Arg Leu Ser Asn Phe
    290                 295                 300

Gln Gly Val Asp Thr Leu Glu Ile Gln Gly Asp Val Thr Leu Ser Tyr
305                 310                 315                 320

Val Gln Ile

<210>  5
<211>  981
<212>  DNA
<213>  Mus musculus

<400>  5
atggcctatg ttccagcacc gggctaccag cccacctaca atccgactct gccctacaag      60

agacccatcc caggtggcct cagtgtcggg atgtccgttt acatccaagg aatggccaaa     120

gagaacatga gacggttcca cgtgaacttc gctgtggggc aggatgatgg ggcggatgtt     180

gctttccact tcaatccccg ctttgatggc tgggacaagg tggtcttcaa cacgatgcaa     240

agtggacaat ggggcaagga agagaagaag aagagcatgc ctttccagaa gggcaagcac     300

ttcgagctgg tgttcatggt catgcctgag cactacaagg tcgtggtgaa cggaaattcc     360

ttctatgaat acgggcaccg gttacctgta cagatggtca cccatctgca agtggatggg     420

gacctggaac ttcagtccat caacttcctc ggaggccaac cagctgcagc ccatatccgg     480

ggagccatga ctattccagc ttaccctgct gggagtcctg ggtacaaccc tccacagatg     540

aacaccttgc ctgtcatgac aggaccccca gtcttcaatc cgcgtgtgcc atatgtgggg     600

gctctgcagg ggggccttac agtccgaaga accatcataa tcaagggata tgtacttcct     660

acagccagga actttgtcat caacttcaag gtgggatcgt ccggagacat agctttacac     720

ctaaaccccc gcataggtga cagtgtggtt cgcaacagct ttatgaatgg ctcttggggc     780

gctgaagaga ggaaggtggc ctacaaccca tttggccctg gcagttcttt tgatctgtca     840

atccgctgcg gcatggatcg attcaaggtg tttgccaatg gccagcacct tttcgacttc 900

tcccatcggt tccaagcatt ccaaatggta gacacgctgg agatcaatgg tgacatcacc 960

ttgtcctacg tccagatctg a 981

<210> 6
<211> 326
<212> PRT
<213> Mus musculus

<400> 6

Met Ala Tyr Val Pro Ala Pro Gly Tyr Gln Pro Thr Tyr Asn Pro Thr
1               5                   10                  15

Leu Pro Tyr Lys Arg Pro Ile Pro Gly Gly Leu Ser Val Gly Met Ser
            20                  25                  30

Val Tyr Ile Gln Gly Met Ala Lys Glu Asn Met Arg Arg Phe His Val
        35                  40                  45

Asn Phe Ala Val Gly Gln Asp Asp Gly Ala Asp Val Ala Phe His Phe
    50                  55                  60

Asn Pro Arg Phe Asp Gly Trp Asp Lys Val Val Phe Asn Thr Met Gln
65                  70                  75                  80

Ser Gly Gln Trp Gly Lys Glu Glu Lys Lys Lys Ser Met Pro Phe Gln
                85                  90                  95

Lys Gly Lys His Phe Glu Leu Val Phe Met Val Met Pro Glu His Tyr
            100                 105                 110

Lys Val Val Val Asn Gly Asn Ser Phe Tyr Glu Tyr Gly His Arg Leu
        115                 120                 125

Pro Val Gln Met Val Thr His Leu Gln Val Asp Gly Asp Leu Glu Leu
    130                 135                 140

Gln Ser Ile Asn Phe Leu Gly Gly Gln Pro Ala Ala Ala Pro Tyr Pro
145                 150                 155                 160

Gly Ala Met Thr Ile Pro Ala Tyr Pro Ala Gly Ser Pro Gly Tyr Asn
                165                 170                 175

Pro Pro Gln Met Asn Thr Leu Pro Val Met Thr Gly Pro Pro Val Phe
            180                 185                 190

Asn Pro Arg Val Pro Tyr Val Gly Ala Leu Gln Gly Gly Leu Thr Val

195                 200                 205

Arg Arg Thr Ile Ile Ile Lys Gly Tyr Val Leu Pro Thr Ala Arg Asn
    210                 215                 220

Phe Val Ile Asn Phe Lys Val Gly Ser Ser Gly Asp Ile Ala Leu His
225                 230                 235                 240

Leu Asn Pro Arg Ile Gly Asp Ser Val Val Arg Asn Ser Phe Met Asn
                245                 250                 255

Gly Ser Trp Gly Ala Glu Glu Arg Lys Val Ala Tyr Asn Pro Phe Gly
            260                 265                 270

Pro Gly Gln Phe Phe Asp Leu Ser Ile Arg Cys Gly Met Asp Arg Phe
        275                 280                 285

Lys Val Phe Ala Asn Gly Gln His Leu Phe Asp Phe Ser His Arg Phe
    290                 295                 300

Gln Ala Phe Gln Met Val Asp Thr Leu Glu Ile Asn Gly Asp Ile Thr
305                 310                 315                 320

Leu Ser Tyr Val Gln Ile
                325

<210>  7
<211>  975
<212>  DNA
<213>  Rattus norvegicus

<400>  7
atggcctatg tcccagcacc cggctaccag cccacctaca atccgacgct gccctacaag      60

aggcccatcc caggtggcct cagtgtcgga atgtccattt acatccaagg aatagccaaa     120

gacaacatga gacggttcca cgtgaacttt gctgtggggc aggatgaggg ggcagatatt     180

gctttccact tcaacccccg gtttgatggc tgggacaagg tggtcttcaa tacgatgcaa     240

agcggacagt ggggcaagga agagaagaag aagagcatgc ctttccaaaa gggtcaccac     300

ttcgagctgg tgttcatggt catgtctgag cactacaagg tcgtggtaaa cggaactccc     360

ttctacgaat atgggcaccg gttgccccta cagatggtca cccacttgca agtggatggg     420

gacctggaac ttcagtcaat caacttcctt gggggccaac ctgctgcatc ccagtatccg     480

ggaaccatga ctattccagc ttatcctagt gctgggtaca accctccaca gatgaacagc     540

ctgcctgtca tggcgggacc cccgatcttc aatccgcctg tgccatatgt ggggaccctg     600

caggggggcc ttacagcccg aagaaccatc ataatcaagg gatatgtact tcctacagcc     660

aagaacctta tcatcaactt caaggtgggg tcgaccggag acatagcttt tcacatgaac 720

ccccgcatag gtgattgtgt ggttcgcaac agctatatga atggctcttg gggctccgaa 780

gagaggaaga taccctacaa cccgtttggc gctgggcagt tctttgatct gtcaatccgc 840

tgcggcacgg atcgattcaa ggtgtttgcc aacggccagc accttttcga cttctcacat 900

cggttccaag cattccaaag ggtagacatg ctggagatca aaggcgacat caccttgtcc 960

tatgtccaga tctga 975

<210> 8
<211> 324
<212> PRT
<213> Rattus norvegicus

<400> 8

Met Ala Tyr Val Pro Ala Pro Gly Tyr Gln Pro Thr Tyr Asn Pro Thr
1               5                   10                  15

Leu Pro Tyr Lys Arg Pro Ile Pro Gly Gly Leu Ser Val Gly Met Ser
            20                  25                  30

Ile Tyr Ile Gln Gly Ile Ala Lys Asp Asn Met Arg Arg Phe His Val
        35                  40                  45

Asn Phe Ala Val Gly Gln Asp Glu Gly Ala Asp Ile Ala Phe His Phe
    50                  55                  60

Asn Pro Arg Phe Asp Gly Trp Asp Lys Val Val Phe Asn Thr Met Gln
65                  70                  75                  80

Ser Gly Gln Trp Gly Lys Glu Glu Lys Lys Lys Ser Met Pro Phe Gln
                85                  90                  95

Lys Gly His His Phe Glu Leu Val Phe Met Val Met Ser Glu His Tyr
            100                 105                 110

Lys Val Val Val Asn Gly Thr Pro Phe Tyr Glu Tyr Gly His Arg Leu
        115                 120                 125

Pro Leu Gln Met Val Thr His Leu Gln Val Asp Gly Asp Leu Glu Leu
    130                 135                 140

Gln Ser Ile Asn Phe Leu Gly Gly Gln Pro Ala Ala Ser Gln Tyr Pro
145                 150                 155                 160

Gly Thr Met Thr Ile Pro Ala Tyr Pro Ser Ala Gly Tyr Asn Pro Pro
                165                 170                 175

Gln Met Asn Ser Leu Pro Val Met Ala Gly Pro Pro Ile Phe Asn Pro
            180                 185                 190

Pro Val Pro Tyr Val Gly Thr Leu Gln Gly Gly Leu Thr Ala Arg Arg
        195                 200                 205

Thr Ile Ile Ile Lys Gly Tyr Val Leu Pro Thr Ala Lys Asn Leu Ile
    210                 215                 220

Ile Asn Phe Lys Val Gly Ser Thr Gly Asp Ile Ala Phe His Met Asn
225                 230                 235                 240

Pro Arg Ile Gly Asp Cys Val Val Arg Asn Ser Tyr Met Asn Gly Ser
                245                 250                 255

Trp Gly Ser Glu Glu Arg Lys Ile Pro Tyr Asn Pro Phe Gly Ala Gly
            260                 265                 270

Gln Phe Phe Asp Leu Ser Ile Arg Cys Gly Thr Asp Arg Phe Lys Val
        275                 280                 285

Phe Ala Asn Gly Gln His Leu Phe Asp Phe Ser His Arg Phe Gln Ala
    290                 295                 300

Phe Gln Arg Val Asp Met Leu Glu Ile Lys Gly Asp Ile Thr Leu Ser
305                 310                 315                 320

Tyr Val Gln Ile

<210>  9
<211>  989
<212>  DNA
<213>  Xenopus laevis

<400>  9
ccaagatggc ttatgtggca gcacccggtt atcagccttt ttacaatcca gccatcccat      60

ttaggactcc tattcatggc ggcctccgtg ctgggatgtc tgtctacatt caggggacta     120

tccccaacca cattaccagg tttgctgtga atttcacttg tggccaattt gatggatcag     180

acattgcctg ccactttaat cctcgctttg atggcaaaga caaggttgtg tttaacagtc     240

ttattggggg ctcctggggg tccgaggaga aaaagaagga ttcctttcct tttcgcaaag     300

ggaagagctt tgacctcgca ttcatgatca accaaagcgc ctttgagatc acagtgaatg     360

gctcttcctt ctataagttc aaacatcgta tgccactgga gcgtgtgaac agtttgcaag     420

tcagtgggga tgtgactgtt cagtccctga ccattgctgg aggtggtggt ggaatgccag     480

cggcccctat gcctgcaccc agtttcccac atccgggggg ggtgatgcca ttaccagttt 540

tcccagctgg caacctacct gtaatgggag gacctgtgta taacccacca gtaccctatt 600

atggctctat ccagggaggc atgtcaccga gaaagacagt gataattaga gggtttattc 660

cagaaggtgc agaaaggttc catataaact ttaaggcagg atcttccaac gatgttgctc 720

tgcattttaa ccctcggttg actgaaaatg tagtggtgag aaacagtcga cttggaggat 780

cctggggaca ggaagaaagg aacctctcgt acaatccatt cagacctgga caatattttg 840

atatttccat acgctgtggg atggatcgct tcacagtttt tgtgaatggg cagcatttct 900

gtgactttgc acacagatat tccatgttcc agatgcttga caggatagaa gttgagggaa 960

atgtggtttt gtctctcgtg cagatctga 989

<210> 10
<211> 327
<212> PRT
<213> Xenopus laevis

<400> 10

Met Ala Tyr Val Ala Ala Pro Gly Tyr Gln Pro Phe Tyr Asn Pro Ala
1               5                   10                  15

Ile Pro Phe Arg Thr Pro Ile His Gly Gly Leu Arg Ala Gly Met Ser
            20                  25                  30

Val Tyr Ile Gln Gly Thr Ile Pro Asn His Ile Thr Arg Phe Ala Val
        35                  40                  45

Asn Phe Thr Cys Gly Gln Phe Asp Gly Ser Asp Ile Ala Cys His Phe
    50                  55                  60

Asn Pro Arg Phe Asp Gly Lys Asp Lys Val Val Phe Asn Ser Leu Ile
65                  70                  75                  80

Gly Gly Ser Trp Gly Ser Glu Glu Lys Lys Lys Asp Ser Phe Pro Phe
                85                  90                  95

Arg Lys Gly Lys Ser Phe Asp Leu Ala Phe Met Ile Asn Gln Ser Ala
            100                 105                 110

Phe Glu Ile Thr Val Asn Gly Ser Ser Phe Tyr Lys Phe Lys His Arg
        115                 120                 125

Met Pro Leu Glu Arg Val Asn Ser Leu Gln Val Ser Gly Asp Val Thr
    130                 135                 140

Val Gln Ser Leu Thr Ile Ala Gly Gly Gly Gly Gly Met Pro Ala Ala

145                 150                 155                 160

Pro Met Pro Ala Pro Ser Phe Pro His Pro Gly Gly Val Met Pro Leu
                165                 170                 175

Pro Val Phe Pro Ala Gly Asn Leu Pro Val Met Gly Gly Pro Val Tyr
            180                 185                 190

Asn Pro Pro Val Pro Tyr Tyr Gly Ser Ile Gln Gly Gly Met Ser Pro
        195                 200                 205

Arg Lys Thr Val Ile Ile Arg Gly Phe Ile Pro Glu Gly Ala Glu Arg
    210                 215                 220

Phe His Ile Asn Phe Lys Ala Gly Ser Ser Asn Asp Val Ala Leu His
225                 230                 235                 240

Phe Asn Pro Arg Leu Thr Glu Asn Val Val Val Arg Asn Ser Arg Leu
                245                 250                 255

Gly Gly Ser Trp Gly Gln Glu Glu Arg Asn Leu Ser Tyr Asn Pro Phe
            260                 265                 270

Arg Pro Gly Gln Tyr Phe Asp Ile Ser Ile Arg Cys Gly Met Asp Arg
        275                 280                 285

Phe Thr Val Phe Val Asn Gly Gln His Phe Cys Asp Phe Ala His Arg
    290                 295                 300

Tyr Ser Met Phe Gln Met Leu Asp Arg Ile Glu Val Glu Gly Asn Val
305                 310                 315                 320

Val Leu Ser Leu Val Gln Ile
                325

<210>  11
<211>  823
<212>  DNA
<213>  Oncorhynchus keta

<400>  11
atgttcgttg ctccacctgg ctaccagccc gtctacaatc ctagtatccc ctatgtgggg      60

ccgatctacg gcgggctgag atcagggatg tctgtataca tccagggagt catccctcac     120

gagatcacca gtttcaacat gaacttgcag tgtggagaat ctgagggcag tgatatcggc     180

tttcacttca gccctcgctt cgacaactgg gacaaggtgg tgttcaacag ctgccaggaa     240

ggggaatggg gttcagagga ggagatccac aacatgcctt ttagcaaggg agacgccttc     300

gagatggtta tcatcattaa gcaggagggt taccaggtgg cagtgaacgg acaagacctt 360

cacacgttca actatcgtat tccagtggag agagtcaacg ccctgcagat aggaggcgac 420

gtttccatcc agaccatcag catcatcggg ggaggaaatc ctggagctga tcaaattggg 480

ggaaacctgc cggtcatgga tggacagaca atattcaaac ctcctgtccc gtactccagc 540

atgattccag gagggatgtc tccgaagaag actatcgtca tcagaggcat ggtgcctcta 600

ggagctaaca gtttctctat taacttcatg gtgggcagct cacgagacat agtgttccac 660

ctaaaccctc gtctacagga gagcgtggtg gtgagaaaca gtttgattgg tgggagctgg 720

gacacggagg agagagagag atcagcctta accccttcct ggagggacag tactttgaca 780

tgtctatccg ttgtgggaac cagcagttca aggtgtttgt gaa 823

<210> 12
<211> 273
<212> PRT
<213> Oncorhynchus keta

<400> 12

Met Phe Val Ala Pro Pro Gly Tyr Gln Pro Val Tyr Asn Pro Ser Ile
1               5                   10                  15

Pro Tyr Val Gly Pro Ile Tyr Gly Gly Leu Arg Ser Gly Met Ser Val
            20                  25                  30

Tyr Ile Gln Gly Val Ile Pro His Glu Ile Thr Ser Phe Asn Met Asn
        35                  40                  45

Leu Gln Cys Gly Glu Ser Glu Gly Ser Asp Ile Gly Phe His Phe Ser
    50                  55                  60

Pro Arg Phe Asp Asn Trp Asp Lys Val Val Phe Asn Ser Cys Gln Glu
65                  70                  75                  80

Gly Glu Trp Gly Ser Glu Glu Glu Ile His Asn Met Pro Phe Ser Lys
                85                  90                  95

Gly Asp Ala Phe Glu Met Val Ile Ile Ile Lys Gln Glu Gly Tyr Gln
            100                 105                 110

Val Ala Val Asn Gly Gln Asp Leu His Thr Phe Asn Tyr Arg Ile Pro
        115                 120                 125

Val Glu Arg Val Asn Ala Leu Gln Ile Gly Gly Asp Val Ser Ile Gln
    130                 135                 140

Thr Ile Ser Ile Ile Gly Gly Gly Asn Pro Gly Ala Asp Gln Ile Gly

145                 150                 155                 160

Gly Asn Leu Pro Val Met Asp Gly Gln Thr Ile Phe Lys Pro Pro Val
                165                 170                 175

Pro Tyr Ser Ser Met Ile Pro Gly Gly Met Ser Pro Lys Lys Thr Ile
            180                 185                 190

Val Ile Arg Gly Met Val Pro Leu Gly Ala Asn Ser Phe Ser Ile Asn
        195                 200                 205

Phe Met Val Gly Ser Ser Arg Asp Ile Val Phe His Leu Asn Pro Arg
    210                 215                 220

Leu Gln Glu Ser Val Val Val Arg Asn Ser Leu Ile Gly Gly Ser Trp
225                 230                 235                 240

Asp Thr Glu Glu Arg Glu Arg Ser Ala Leu Thr Pro Ser Trp Arg Asp
                245                 250                 255

Ser Thr Leu Thr Cys Leu Ser Val Val Gly Thr Ser Ser Ser Arg Cys
            260                 265                 270

Leu

<210>  13
<211>  906
<212>  DNA
<213>  Mus musculus

<400>  13
atggcctatg ttccagcacc gggctaccag cccacctaca atccgactct gccctacaag      60

agacccatcc caggtggcct cagtgtcggg atgtcctttt acatccaagg aacggccaaa     120

gagaacatga gacggttcca cgtgaacttc gctgtggggc aggatgatgg ggcggatgtt     180

gctttccact tcaatccccg ctttgatggc tgggacaagg tggtcttcaa cacgaagcaa     240

agcggacgat ggggcaagga agaggagaag agcatgcctt tccagaaggg caagcacttc     300

gagctggtgt tcatggtcat gcctgagcac tacaaggtcg tggtgaatgg aagtcccttc     360

tatgaatacg ggcaccggtt acccgtacag atggtcaccc atctgcaagt ggatggggac     420

ctggaacttc agtccatcaa cttctttgga gtccaacctg ctgaaaccaa atatccggcc     480

atgacaggac ccccagtctt caatccgtgt ctgccatatg tgggggctct gcaggggggc     540

tttacagtcc gaagaaccat cataatcaag ggatatgtac ttcctacagc caagaccttt     600

gccatcaact tcagggtggg atcttccgaa gacatagctt tacacataaa cccccgcata     660

ggtgactgtt tggttcgcaa cagctatatg aatggctctt ggggcactga agagaggatg 720

gtggcctaca acccatttgg ccctgggcag ttctttgatc tgtcaatccg ctgcggcatg 780

gatcgattca aggtgtttgc caatggcatt caccttttca acttctccca tcggttccaa 840

gctttacgaa agataaacac gctggagatc aatggtgacc tcaccttgtc ctacgtccac 900

atctga 906

<210> 14
<211> 301
<212> PRT
<213> Mus musculus

<400> 14

Met Ala Tyr Val Pro Ala Pro Gly Tyr Gln Pro Thr Tyr Asn Pro Thr
1               5                   10                  15

Leu Pro Tyr Lys Arg Pro Ile Pro Gly Gly Leu Ser Val Gly Met Ser
            20                  25                  30

Phe Tyr Ile Gln Gly Thr Ala Lys Glu Asn Met Arg Arg Phe His Val
        35                  40                  45

Asn Phe Ala Val Gly Gln Asp Asp Gly Ala Asp Val Ala Phe His Phe
    50                  55                  60

Asn Pro Arg Phe Asp Gly Trp Asp Lys Val Val Phe Asn Thr Lys Gln
65                  70                  75                  80

Ser Gly Arg Trp Gly Lys Glu Glu Glu Lys Ser Met Pro Phe Gln Lys
                85                  90                  95

Gly Lys His Phe Glu Leu Val Phe Met Val Met Pro Glu His Tyr Lys
            100                 105                 110

Val Val Val Asn Gly Ser Pro Phe Tyr Glu Tyr Gly His Arg Leu Pro
        115                 120                 125

Val Gln Met Val Thr His Leu Gln Val Asp Gly Asp Leu Glu Leu Gln
    130                 135                 140

Ser Ile Asn Phe Phe Gly Val Gln Pro Ala Glu Thr Lys Tyr Pro Ala
145                 150                 155                 160

Met Thr Gly Pro Pro Val Phe Asn Pro Cys Leu Pro Tyr Val Gly Ala
                165                 170                 175

Leu Gln Gly Gly Phe Thr Val Arg Arg Thr Ile Ile Ile Lys Gly Tyr

180                 185                 190

Val Leu Pro Thr Ala Lys Thr Phe Ala Ile Asn Phe Arg Val Gly Ser
        195                 200                 205

Ser Glu Asp Ile Ala Leu His Ile Asn Pro Arg Ile Gly Asp Cys Leu
    210                 215                 220

Val Arg Asn Ser Tyr Met Asn Gly Ser Trp Gly Thr Glu Glu Arg Met
225                 230                 235                 240

Val Ala Tyr Asn Pro Phe Gly Pro Gly Gln Phe Phe Asp Leu Ser Ile
                245                 250                 255

Arg Cys Gly Met Asp Arg Phe Lys Val Phe Ala Asn Gly Ile His Leu
            260                 265                 270

Phe Asn Phe Ser His Arg Phe Gln Ala Leu Arg Lys Ile Asn Thr Leu
        275                 280                 285

Glu Ile Asn Gly Asp Leu Thr Leu Ser Tyr Val His Ile
    290                 295                 300

<210>  15
<211>  3768
<212>  DNA
<213>  Homo sapiens

<400>  15
atgacaccgg gcacccagtc tcctttcttc ctgctgctgc tcctcacagt gcttacagtt      60

gttacaggtt ctggtcatgc aagctctacc ccaggtggag aaaaggagac ttcggctacc     120

cagagaagtt cagtgcccag ctctactgag aagaatgctg tgagtatgac cagcagcgta     180

ctctccagcc acagccccgg ttcaggctcc tccaccactc agggacagga tgtcactctg     240

gccccggcca cggaaccagc ttcaggttca gctgccacct ggggacagga tgtcacctcg     300

gtcccagtca ccaggccagc cctgggctcc accaccccgc cagcccacga tgtcacctca     360

gccccggaca acaagccagc cccgggctcc accgcccccc cagcccacgg tgtcacctcg     420

gccccggaca ccaggccggc cccgggctcc accgcccccc cagcccacgg tgtcacctcg     480

gccccggaca ccaggccggc cccgggctcc accgcccccc cagcccacgg tgtcacctcg     540

gccccggaca ccaggccggc cccgggctcc accgcccccc cagcccacgg tgtcacctcg     600

gccccggaca ccaggccggc cccgggctcc accgcccccc cagcccacgg tgtcacctcg     660

gccccggaca ccaggccggc cccgggctcc accgcccccc cagcccacgg tgtcacctcg     720

gccccggaca ccaggccggc cccgggctcc accgcccccc cagcccacgg tgtcacctcg     780

gccccggaca ccaggccggc cccgggctcc accgcccccc cagcccacgg tgtcacctcg 840
gccccggaca ccaggccggc cccgggctcc accgcccccc cagcccacgg tgtcacctcg 900
gccccggaca ccaggccggc cccgggctcc accgcccccc cagcccacgg tgtcacctcg 960
gccccggaca ccaggccggc cccgggctcc accgcccccc cagcccacgg tgtcacctcg 1020
gccccggaca ccaggccggc cccgggctcc accgcccccc cagcccacgg tgtcacctcg 1080
gccccggaca ccaggccggc cccgggctcc accgcccccc cagcccacgg tgtcacctcg 1140
gccccggaca ccaggccggc cccgggctcc accgcccccc cagcccacgg tgtcacctcg 1200
gccccggaca ccaggccggc cccgggctcc accgcccccc cagcccacgg tgtcacctcg 1260
gccccggaca ccaggccggc cccgggctcc accgcccccc cagcccacgg tgtcacctcg 1320
gccccggaca ccaggccggc cccgggctcc accgcccccc cagcccacgg tgtcacctcg 1380
gccccggaca ccaggccggc cccgggctcc accgcccccc cagcccacgg tgtcacctcg 1440
gccccggaca ccaggccggc cccgggctcc accgcccccc cagcccacgg tgtcacctcg 1500
gccccggaca ccaggccggc cccgggctcc accgcccccc cagcccacgg tgtcacctcg 1560
gccccggaca ccaggccggc cccgggctcc accgcccccc cagcccacgg tgtcacctcg 1620
gccccggaca ccaggccggc cccgggctcc accgcccccc cagcccacgg tgtcacctcg 1680
gccccggaca ccaggccggc cccgggctcc accgcccccc cagcccacgg tgtcacctcg 1740
gccccggaca ccaggccggc cccgggctcc accgcccccc cagcccacgg tgtcacctcg 1800
gccccggaca ccaggccggc cccgggctcc accgcccccc cagcccacgg tgtcacctcg 1860
gccccggaca ccaggccggc cccgggctcc accgcccccc cagcccacgg tgtcacctcg 1920
gccccggaca ccaggccggc cccgggctcc accgcccccc cagcccacgg tgtcacctcg 1980
gccccggaca ccaggccggc cccgggctcc accgcccccc cagcccacgg tgtcacctcg 2040
gccccggaca ccaggccggc cccgggctcc accgcccccc cagcccacgg tgtcacctcg 2100
gccccggaca ccaggccggc cccgggctcc accgcccccc cagcccacgg tgtcacctcg 2160
gccccggaca ccaggccggc cccgggctcc accgcccccc cagcccacgg tgtcacctcg 2220
gccccggaca ccaggccggc cccgggctcc accgcccccc cagcccacgg tgtcacctcg 2280
gccccggaca ccaggccggc cccgggctcc accgcccccc cagcccacgg tgtcacctcg 2340
gccccggaca ccaggccggc cccgggctcc accgcccccc cagcccacgg tgtcacctcg 2400
gccccggaca ccaggccggc cccgggctcc accgcccccc cagcccacgg tgtcacctcg 2460
gccccggaca ccaggccggc cccgggctcc accgcccccc cagcccacgg tgtcacctcg 2520
gccccggaca ccaggccggc cccgggctcc accgcccccc cagcccacgg tgtcacctcg 2580
gccccggaca ccaggccggc cccgggctcc accgcccccc cagcccacgg tgtcacctcg 2640
gccccggaca ccaggccggc cccgggctcc accgcccccc cagcccacgg tgtcacctcg 2700

gccccggaca ccaggccggc cccgggctcc accgcccccc cagcccacgg tgtcacctcg 2760

gccccggaca ccaggccggc cccgggctcc accgcccccc cagcccatgg tgtcacctcg 2820

gccccggaca acaggcccgc cttgggctcc accgcccctc cagtccacaa tgtcacctcg 2880

gcctcaggct ctgcatcagg ctcagcttct actctggtgc acaacggcac ctctgccagg 2940

gctaccacaa ccccagccag caagagcact ccattctcaa ttcccagcca ccactctgat 3000

actcctacca cccttgccag ccatagcacc aagactgatg ccagtagcac tcaccatagc 3060

tcggtacctc ctctcacctc ctccaatcac agcacttctc cccagttgtc tactggggtc 3120

tctttctttt tcctgtcttt tcacatttca aacctccagt ttaattcctc tctggaagat 3180

cccagcaccg actactacca agagctgcag agagacattt ctgaaatgtt tttgcagatt 3240

tataaacaag ggggttttct gggcctctcc aatattaagt tcaggccagg atctgtggtg 3300

gtacaattga ctctggcctt ccgagaaggt accatcaatg tccacgacgt ggagacacag 3360

ttcaatcagt ataaaacgga agcagcctct cgatataacc tgacgatctc agacgtcagc 3420

gtgagtgatg tgccatttcc tttctctgcc cagtctgggg ctggggtgcc aggctggggc 3480

atcgcgctgc tggtgctggt ctgtgttctg gttgcgctgg ccattgtcta tctcattgcc 3540

ttggctgtct gtcagtgccg ccgaaagaac tacgggcagc tggacatctt tccagcccgg 3600

gatacctacc atcctatgag cgagtacccc acctaccaca cccatgggcg ctatgtgccc 3660

cctagcagta ccgatcgtag cccctatgag aaggtttctg caggtaacgg tggcagcagc 3720

ctctcttaca caaacccagc agtggcagcc gcttctgcca acttgtag 3768

<210> 16
<211> 1255
<212> PRT
<213> Homo sapiens

<400> 16

Met Thr Pro Gly Thr Gln Ser Pro Phe Phe Leu Leu Leu Leu Leu Thr
1               5                   10                  15

Val Leu Thr Val Val Thr Gly Ser Gly His Ala Ser Ser Thr Pro Gly
            20                  25                  30

Gly Glu Lys Glu Thr Ser Ala Thr Gln Arg Ser Ser Val Pro Ser Ser
        35                  40                  45

Thr Glu Lys Asn Ala Val Ser Met Thr Ser Ser Val Leu Ser Ser His
    50                  55                  60

Ser Pro Gly Ser Gly Ser Ser Thr Thr Gln Gly Gln Asp Val Thr Leu
65                  70                  75                  80

Ala Pro Ala Thr Glu Pro Ala Ser Gly Ser Ala Ala Thr Trp Gly Gln
                85                  90                  95

Asp Val Thr Ser Val Pro Val Thr Arg Pro Ala Leu Gly Ser Thr Thr
            100                 105                 110

Pro Pro Ala His Asp Val Thr Ser Ala Pro Asp Asn Lys Pro Ala Pro
        115                 120                 125

Gly Ser Thr Ala Pro Pro Ala His Gly Val Thr Ser Ala Pro Asp Thr
    130                 135                 140

Arg Pro Ala Pro Gly Ser Thr Ala Pro Pro Ala His Gly Val Thr Ser
145                 150                 155                 160

Ala Pro Asp Thr Arg Pro Ala Pro Gly Ser Thr Ala Pro Pro Ala His
                165                 170                 175

Gly Val Thr Ser Ala Pro Asp Thr Arg Pro Ala Pro Gly Ser Thr Ala
            180                 185                 190

Pro Pro Ala His Gly Val Thr Ser Ala Pro Asp Thr Arg Pro Ala Pro
        195                 200                 205

Gly Ser Thr Ala Pro Pro Ala His Gly Val Thr Ser Ala Pro Asp Thr
    210                 215                 220

Arg Pro Ala Pro Gly Ser Thr Ala Pro Pro Ala His Gly Val Thr Ser
225                 230                 235                 240

Ala Pro Asp Thr Arg Pro Ala Pro Gly Ser Thr Ala Pro Pro Ala His
                245                 250                 255

Gly Val Thr Ser Ala Pro Asp Thr Arg Pro Ala Pro Gly Ser Thr Ala
            260                 265                 270

Pro Pro Ala His Gly Val Thr Ser Ala Pro Asp Thr Arg Pro Ala Pro
        275                 280                 285

Gly Ser Thr Ala Pro Pro Ala His Gly Val Thr Ser Ala Pro Asp Thr
    290                 295                 300

Arg Pro Ala Pro Gly Ser Thr Ala Pro Pro Ala His Gly Val Thr Ser
305                 310                 315                 320

Ala Pro Asp Thr Arg Pro Ala Pro Gly Ser Thr Ala Pro Pro Ala His

325                 330                 335

Gly Val Thr Ser Ala Pro Asp Thr Arg Pro Ala Pro Gly Ser Thr Ala
            340                 345                 350

Pro Pro Ala His Gly Val Thr Ser Ala Pro Asp Thr Arg Pro Ala Pro
        355                 360                 365

Gly Ser Thr Ala Pro Pro Ala His Gly Val Thr Ser Ala Pro Asp Thr
    370                 375                 380

Arg Pro Ala Pro Gly Ser Thr Ala Pro Pro Ala His Gly Val Thr Ser
385                 390                 395                 400

Ala Pro Asp Thr Arg Pro Ala Pro Gly Ser Thr Ala Pro Pro Ala His
                405                 410                 415

Gly Val Thr Ser Ala Pro Asp Thr Arg Pro Ala Pro Gly Ser Thr Ala
            420                 425                 430

Pro Pro Ala His Gly Val Thr Ser Ala Pro Asp Thr Arg Pro Ala Pro
        435                 440                 445

Gly Ser Thr Ala Pro Pro Ala His Gly Val Thr Ser Ala Pro Asp Thr
    450                 455                 460

Arg Pro Ala Pro Gly Ser Thr Ala Pro Pro Ala His Gly Val Thr Ser
465                 470                 475                 480

Ala Pro Asp Thr Arg Pro Ala Pro Gly Ser Thr Ala Pro Pro Ala His
                485                 490                 495

Gly Val Thr Ser Ala Pro Asp Thr Arg Pro Ala Pro Gly Ser Thr Ala
            500                 505                 510

Pro Pro Ala His Gly Val Thr Ser Ala Pro Asp Thr Arg Pro Ala Pro
        515                 520                 525

Gly Ser Thr Ala Pro Pro Ala His Gly Val Thr Ser Ala Pro Asp Thr
    530                 535                 540

Arg Pro Ala Pro Gly Ser Thr Ala Pro Pro Ala His Gly Val Thr Ser
545                 550                 555                 560

Ala Pro Asp Thr Arg Pro Ala Pro Gly Ser Thr Ala Pro Pro Ala His
                565                 570                 575

Gly Val Thr Ser Ala Pro Asp Thr Arg Pro Ala Pro Gly Ser Thr Ala
            580                 585                 590

Pro Pro Ala His Gly Val Thr Ser Ala Pro Asp Thr Arg Pro Ala Pro
        595                 600                 605

Gly Ser Thr Ala Pro Pro Ala His Gly Val Thr Ser Ala Pro Asp Thr
    610                 615                 620

Arg Pro Ala Pro Gly Ser Thr Ala Pro Pro Ala His Gly Val Thr Ser
625                 630                 635                 640

Ala Pro Asp Thr Arg Pro Ala Pro Gly Ser Thr Ala Pro Pro Ala His
                645                 650                 655

Gly Val Thr Ser Ala Pro Asp Thr Arg Pro Ala Pro Gly Ser Thr Ala
            660                 665                 670

Pro Pro Ala His Gly Val Thr Ser Ala Pro Asp Thr Arg Pro Ala Pro
        675                 680                 685

Gly Ser Thr Ala Pro Pro Ala His Gly Val Thr Ser Ala Pro Asp Thr
    690                 695                 700

Arg Pro Ala Pro Gly Ser Thr Ala Pro Pro Ala His Gly Val Thr Ser
705                 710                 715                 720

Ala Pro Asp Thr Arg Pro Ala Pro Gly Ser Thr Ala Pro Pro Ala His
                725                 730                 735

Gly Val Thr Ser Ala Pro Asp Thr Arg Pro Ala Pro Gly Ser Thr Ala
            740                 745                 750

Pro Pro Ala His Gly Val Thr Ser Ala Pro Asp Thr Arg Pro Ala Pro
        755                 760                 765

Gly Ser Thr Ala Pro Pro Ala His Gly Val Thr Ser Ala Pro Asp Thr
    770                 775                 780

Arg Pro Ala Pro Gly Ser Thr Ala Pro Pro Ala His Gly Val Thr Ser
785                 790                 795                 800

Ala Pro Asp Thr Arg Pro Ala Pro Gly Ser Thr Ala Pro Pro Ala His
                805                 810                 815

Gly Val Thr Ser Ala Pro Asp Thr Arg Pro Ala Pro Gly Ser Thr Ala
            820                 825                 830

Pro Pro Ala His Gly Val Thr Ser Ala Pro Asp Thr Arg Pro Ala Pro
        835                 840                 845

Gly Ser Thr Ala Pro Pro Ala His Gly Val Thr Ser Ala Pro Asp Thr
    850                 855                 860

Arg Pro Ala Pro Gly Ser Thr Ala Pro Pro Ala His Gly Val Thr Ser
865                 870                 875                 880

Ala Pro Asp Thr Arg Pro Ala Pro Gly Ser Thr Ala Pro Pro Ala His
                885                 890                 895

Gly Val Thr Ser Ala Pro Asp Thr Arg Pro Ala Pro Gly Ser Thr Ala
            900                 905                 910

Pro Pro Ala His Gly Val Thr Ser Ala Pro Asp Thr Arg Pro Ala Pro
        915                 920                 925

Gly Ser Thr Ala Pro Pro Ala His Gly Val Thr Ser Ala Pro Asp Asn
    930                 935                 940

Arg Pro Ala Leu Gly Ser Thr Ala Pro Pro Val His Asn Val Thr Ser
945                 950                 955                 960

Ala Ser Gly Ser Ala Ser Gly Ser Ala Ser Thr Leu Val His Asn Gly
                965                 970                 975

Thr Ser Ala Arg Ala Thr Thr Thr Pro Ala Ser Lys Ser Thr Pro Phe
            980                 985                 990

Ser Ile Pro Ser His His Ser Asp Thr Pro Thr Thr Leu Ala Ser His
        995                 1000                1005

Ser Thr Lys Thr Asp Ala Ser Ser Thr His His Ser Ser Val Pro
    1010                1015                1020

Pro Leu Thr Ser Ser Asn His Ser Thr Ser Pro Gln Leu Ser Thr
    1025                1030                1035

Gly Val Ser Phe Phe Phe Leu Ser Phe His Ile Ser Asn Leu Gln
    1040                1045                1050

Phe Asn Ser Ser Leu Glu Asp Pro Ser Thr Asp Tyr Tyr Gln Glu
    1055                1060                1065

Leu Gln Arg Asp Ile Ser Glu Met Phe Leu Gln Ile Tyr Lys Gln
    1070                1075                1080

Gly Gly Phe Leu Gly Leu Ser Asn Ile Lys Phe Arg Pro Gly Ser
    1085                1090                1095

Val Val Val Gln Leu Thr Leu Ala Phe Arg Glu Gly Thr Ile Asn
    1100                1105                1110

Val His Asp Val Glu Thr Gln Phe Asn Gln Tyr Lys Thr Glu Ala
    1115                1120                1125

Ala Ser Arg Tyr Asn Leu Thr Ile Ser Asp Val Ser Val Ser Asp
    1130                1135                1140

Val Pro Phe Pro Phe Ser Ala Gln Ser Gly Ala Gly Val Pro Gly
    1145                1150                1155

Trp Gly Ile Ala Leu Leu Val Leu Val Cys Val Leu Val Ala Leu
    1160                1165                1170

Ala Ile Val Tyr Leu Ile Ala Leu Ala Val Cys Gln Cys Arg Arg
    1175                1180                1185

Lys Asn Tyr Gly Gln Leu Asp Ile Phe Pro Ala Arg Asp Thr Tyr
    1190                1195                1200

His Pro Met Ser Glu Tyr Pro Thr Tyr His Thr His Gly Arg Tyr
    1205                1210                1215

Val Pro Pro Ser Ser Thr Asp Arg Ser Pro Tyr Glu Lys Val Ser
    1220                1225                1230

Ala Gly Asn Gly Gly Ser Ser Leu Ser Tyr Thr Asn Pro Ala Val
    1235                1240                1245

Ala Ala Ala Ser Ala Asn Leu
    1250                1255

<210> 17
<211> 27
<212> DNA
<213> Artificial

<220>
<223> primer for human galectin-4

<400> 17
gctgtcgaca tggcctatgt ccccgca 27

<210> 18

<211>  27
<212>  DNA
<213>  Artificial

<220>
<223>  primer for human galectin-4

<400>  18
cctaagcttt ctggacatag gacaagg                                          27

## Description

### Technical Field

The present invention relates to a method for analyzing a mucin 1 having 3' sulfonated Galβ1-3GalNAc carbohydrate chain (hereinbelow, it may be referred to as 3' sulfonated core 1 carbohydrate chain); a method for detecting or monitoring breast cancer using the method; a kit for analyzing a mucin 1 having a 3' sulfonated core 1 carbohydrate chain, and; a kit for detecting or monitoring breast cancer.

### Background Art

Breast cancer is the fourth most common type of cancer affecting women. However, in a limited age range of 30 to 65 years, it is the most common type of cancer affecting women. In particular, more Japanese women have been diagnosed with breast cancer in recent years, and it is estimated that at least 40,000 new patients are found each year.

CA15-3 as a clinical laboratory test item is used as a clinical marker of breast cancer. It is useful as an index of malignancy and degree of the progress of breast cancer, as well as monitoring the therapeutic effects against breast cancer (Non Patent Literature 1). CA15-3 is detected by an immunoassay method (sandwich method) using a monoclonal antibody (115D8) (Non Patent Literature 2) against MAM-6, which is a glycoprotein on the human milk fat globule membrane, and a monoclonal antibody (DF3) (Non Patent Literature 3) against liver tissue to which breast cancer spreads by metastasis. The antigen detected by the CA15-3 immunoassay method is an extracellular domain of a mucin 1 derived from an epithelial cell.

However, although CA15-3 is useful for monitoring an effect in treatment of breast cancer, it has poor sensitivity, resulting in a positive ratio as low as 15% among all breast cancer patients. Therefore, its use has been limited.

### Citation List

### Non Patent Literatures

Non Patent Literature 1: Breast Cancer (Japan) 2003, Vol. 10, p. 38-44
Non Patent Literature 2: International Journal of Cancer (USA) 1984, Vol. 34, p. 197-206
Non Patent Literature 3: Hybridoma (USA) 1984, Vol. 3, p. 223-232
Non Patent Literature 4: Glycobiology (USA) 2002, Vol. 12, p. 199-208
Non Patent Literature 5: Journal of American Chemical Society (USA) 2009, Vol. 131, p. 17102-17109

### Summary of Invention

### Technical Problem

As described above, although it is useful as an index of malignancy and degree of progress of breast cancer, and for monitoring the therapeutic effects against breast cancer, CA15-3 has the issue of having a low positive ratio.

An object of the present invention is to provide a method for analyzing a mucin 1 derived from breast cancer that cannot be detected by a CA15-3 measurement method. It is also to provide a marker for monitoring malignancy and the degree of progress of breast cancer, and a marker for monitoring therapeutic effects. It is also to provide a method of analyzing a mucin 1 present in an early stage breast cancer patient from which CA15-3 cannot be detected.

### Solution to Problem

The inventors of the present invention conducted intensive studies on a mucin 1 secreted from breast cancer cells, and as a result, surprisingly found that a mucin 1 of a breast cancer patient has a Sulfo-3Galβ1-3GalNAc-R carbohydrate chain (3' sulfonated core 1 carbohydrate chain), which is a carbohydrate chain antigen. By using a probe capable of recognizing the mucin 1 having a 3' sulfonated core 1 carbohydrate chain, the inventors of the present invention found an analysis method having a better detection rate than the CA15-3 measurement method. It was also found that, by using the analysis method of the present invention, a mucin 1 of a breast cancer patient, which cannot be detected by the CA15-3 measurement method, can be detected. Because the ELISA using lectin of a related art has low affinity for the carbohydrate chain of lectin, many of them have low sensitivity. The analysis method of the present invention using lectin bound with a 3' sulfonated core 1 carbohydrate chain has very high sensitivity, and the present invention is surprising from such a point of view.

The present invention is based on those findings.

Specifically, the present invention is:
[1] a method for analyzing a mucin 1 having a Sulfo-3Galβ1-3GalNAc-R carbohydrate chain, the method including:
   (A) a step of bringing a 3' sulfonated core 1 carbohydrate chain binding probe binding to a Sulfo-3Galβ1-3GalNAc-R carbohydrate chain into contact with a sample to be tested;
      a step of bringing a 3' sulfonated core 1 carbohydrate chain mucin 1 peptide binding probe binding to a mucin 1 having a Sulfo-3Galβ1-3GalNAc-R carbohydrate chain or a mucin 1 binding probe binding to a mucin 1 into contact with the sample to be tested; and
      a step of detecting a conjugate of the mucin 1 having a Sulfo-3Galβ1-3GalNAc-R carbohydrate chain and the probe; or
   (B) a step of bringing a 3' sulfonated core 1 carbohydrate chain mucin 1 peptide binding probe binding to a mucin 1 having a Sulfo-3Galβ1-3GalNAc-R carbohydrate chain into contact with a sample to be tested;
      a step of bringing a 3' sulfonated core 1 carbohydrate chain mucin 1 peptide binding probe binding to the mucin 1 having a Sulfo-3Galβ1-3GalNAc-R carbohydrate chain, a mucin 1 binding probe binding to a mucin 1, or a mucin 1 carbohydrate chain binding probe binding to a carbohydrate chain possessed by mucin 1 into contact with the sample to be tested; and
      a step of detecting a conjugate of the mucin 1 having a Sulfo-3Galβ1-3GalNAc-R carbohydrate chain and the probe;
[2] a method for analyzing a mucin 1 having a Sulfo-3Galβ1-3GalNAc-R carbohydrate chain, the method including a step of bringing a 3' sulfonated core 1 carbohydrate chain binding probe binding to a Sulfo-3Galβ1-3GalNAc-R carbohydrate chain into contact with a sample to be tested;
[3] the method for analyzing a mucin 1 having a Sulfo-3Galβ1-3GalNAc-R carbohydrate chain according to claim 2, the method including:
   a step of bringing a 3' sulfonated core 1 carbohydrate chain binding probe binding to a Sulfo-3Galβ1-3GalNAc-R carbohydrate chain into contact with a sample to be tested; and
   a step of detecting a conjugate of the mucin 1 having a Sulfo-3Galβ1-3GalNAc-R carbohydrate chain and the probe;
[4] the method for analyzing a mucin 1 having a Sulfo-3Galβ1-3GalNAc-R carbohydrate chain according to any of [1] to [3], wherein the 3' sulfonated core 1 carbohydrate chain binding probe is a 3' sulfonated core 1 carbohydrate chain binding lectin or a lectin fragment having a carbohydrate chain binding region thereof, or a 3' sulfonated core 1 carbohydrate chain binding antibody or an antibody fragment having an antigen binding region thereof, the 3' sulfonated core 1 carbohydrate chain mucin 1 peptide binding probe is a 3' sulfonated core 1 carbohydrate chain mucin 1 peptide binding antibody or an antibody fragment having an antigen binding region thereof, the mucin 1 binding probe is a mucin 1 peptide binding antibody or an antibody fragment having an antigen binding region thereof, or a mucin 1 carbohydrate chain peptide binding antibody or an antibody fragment having an antigen binding region thereof, and the mucin 1 carbohydrate chain binding probe is a mucin 1 carbohydrate chain binding lectin or a lectin fragment having a carbohydrate chain binding region thereof, or a mucin 1 carbohydrate chain binding antibody or an antibody fragment having an antigen binding region thereof;
[5] the method for analyzing a mucin 1 having a Sulfo-3Galβ1-3GalNAc-R carbohydrate chain according to [4], wherein the 3' sulfonated core 1 carbohydrate chain binding lectin is selected from the group consisting of galectin-1, galectin-3, galectin-4, galectin-6, galectin-8, and galectin-9;
[6] the method for analyzing a mucin 1 having a Sulfo-3Galβ1-3GalNAc-R carbohydrate chain according to [5], wherein the galectin-4 is galectin-4 originated from mammals, birds, amphibians, reptiles, or fishes;
[7] a method for detecting or monitoring breast cancer, the method including analyzing an amount of the mucin 1 having a Sulfo-3Galβ1-3GalNAc-R carbohydrate chain according to the analysis method described in any of [1] to [6];
[8] a kit for analyzing a mucin 1 having a Sulfo-3Galβ1-3GalNAc-R carbohydrate chain, the kit including a 3' sulfonated core 1 carbohydrate chain binding probe binding to a Sulfo-3Galβ1-3GalNAc-R carbohydrate chain;
[9] a kit for analyzing a mucin 1 having a Sulfo-3Galβ1-3GalNAc-R carbohydrate chain, the kit including:
   (A) a 3' sulfonated core 1 carbohydrate chain binding probe binding to a Sulfo-3Galβ1-3GalNAc-R carbohydrate chain; and a 3' sulfonated core 1 carbohydrate chain mucin 1 peptide binding probe binding to a mucin 1 having a Sulfo-3Galβ1-3GalNAc-R carbohydrate chain or a mucin 1 binding probe binding to a mucin 1, or
   (B) a 3' sulfonated core 1 carbohydrate chain mucin 1 peptide binding probe binding to a mucin 1 having a Sulfo-3Galβ1-3GalNAc-R carbohydrate chain; and
      a mucin 1 binding probe binding to a mucin 1, or a mucin 1 carbohydrate chain binding probe binding to a carbohydrate chain possessed by mucin 1;
[10] the kit for analyzing a mucin 1 having a Sulfo-3Galβ1-3GalNAc-R according to [8] or [9], wherein the 3' sulfonated core 1 carbohydrate chain binding probe is a 3' sulfonated core 1 carbohydrate chain binding lectin or a lectin fragment having a carbohydrate chain binding region thereof, or a 3' sulfonated core 1 carbohydrate chain binding antibody or an antibody fragment having an antigen binding region thereof, the 3' sulfonated core 1 carbohydrate chain mucin 1 peptide binding probe is a 3' sulfonated core 1 carbohydrate chain mucin 1 peptide binding antibody or an antibody fragment having an antigen binding region thereof, the mucin 1 binding probe is a mucin 1 peptide binding antibody or an antibody fragment having an antigen binding region thereof, or a mucin 1 carbohydrate chain peptide binding antibody or an antibody fragment having an antigen binding region thereof, and the mucin 1 carbohydrate chain binding probe is a mucin 1 carbohydrate chain binding lectin or a lectin fragment having a carbohydrate chain binding region thereof, or a mucin 1 carbohydrate chain binding antibody or an antibody fragment having an antigen binding region thereof;
[11] the kit for analyzing a mucin 1 having a Sulfo-3Galβ1-3GalNAc-R according to [10], wherein the 3' sulfonated core 1 carbohydrate chain binding lectin is selected from the group consisting of galectin-1, galectin-3, galectin-4, galectin-6, galectin-8, and galectin-9;
[12] the kit for analyzing a mucin 1 having a Sulfo-3Galβ1-3GalNAc-R according to [11], wherein the galectin-4 is galectin-4 originated from mammals, birds, amphibians, reptiles, or fishes;
[13] the kit for analyzing a mucin 1 having a Sulfo-3Galβ1-3GalNAc-R carbohydrate chain according to any of [8] to [12], the kit further including a mucin 1 having a Sulfo-3Galβ1-3GalNAc-R carbohydrate chain;
[14] a kit for detecting or monitoring breast cancer using the kit for analyzing a mucin 1 having a Sulfo-3Galβ1-3GalNAc-R carbohydrate chain described in any of [8] to [13]; and
[15] a mucin 1 having a Sulfo-3Galβ1-3GalNAc-R carbohydrate chain.

Meanwhile, the term "analysis" as used herein includes both "measurement" for a quantitative or semiquantitative determination of an amount of a subject material for analysis and "detection" for determining the presence or the absence of a subject material for analysis.

### Advantageous Effects of Invention

With the analysis method or analysis kit for the mucin 1 having a Sulfo-3Galβ1-3GalNAc-R carbohydrate chain of the present invention, a mucin 1 of a breast cancer patient can be detected at a high rate. Further, with the analysis method or analysis kit of the present invention, it is possible to predict recurrence or metastasis in a breast cancer patient, and thus it can be used as a marker for recurrence or metastasis of breast cancer.

### Brief Description of Drawings

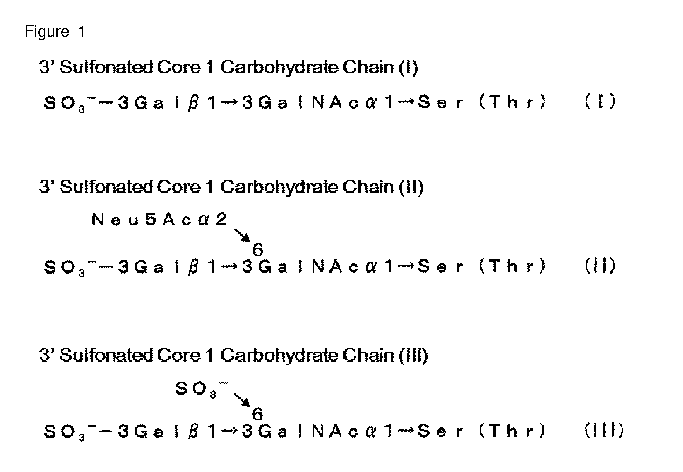

Fig. 1 is a drawing illustrating a major 3' sulfonated Galβ1-3GalNAc carbohydrate chain of a mucin 1.
Fig. 2 is a drawing illustrating the sequence homology between the amino acid sequence of galectin-4 of a human, a pig, a mouse, a rat, a *Xenopus laevis,* or a salmon and the amino sequence of galectin-6 of a mouse.
Fig. 3 is a drawing illustrating the calibration curve for measurement of the mucin 1 having a 3' sulfonated core 1 carbohydrate chain via the sandwich method.
Fig. 4 is a drawing illustrating a graph in which a measurement value of the mucin 1 having a 3' sulfonated core 1 carbohydrate chain in a serum of a breast cancer patient with recurrence or metastasis or a healthy subject and measurement value of CA15-3 in a serum of a breast cancer patient with recurrence are plotted.
Fig. 5 is a drawing illustrating the relation between the measurement value of the mucin 1 having a 3' sulfonated core 1 carbohydrate chain and the measurement value of CA15-3.
Fig. 6 is a drawing illustrating the measurement value of the mucin 1 having a 3' sulfonated core 1 carbohydrate chain from a breast cancer patient with recurrence, a breast cancer patient with no indication of recurrence over 5 years or longer after surgery, or a healthy subject.
Fig. 7 is a graph in which the relation between measurement values of CA15-3 and Gal4/MUC1 in a patient with primary breast cancer at stage I and stage II is plotted.

### Description of Embodiments

### [1] A mucin 1 having a Sulfo-3Galβ1-3GalNAc-R carbohydrate chain

A mucin 1 is a highly glycosylated type I membrane glycoprotein having a molecular weight of approximately 300 kDa or more, which consists of a short N-terminal region, a center region, a transmembrane region, and a C-terminal cytoplasmic region. The center region contains a unique tandem repeat consisting of twenty amino acids (PDTRPAPGSTAPPAHGVTSA). A mucin 1 gene is a variable, and thus the number of the region encoding tandem repeats varies from 25 to 125. Further, a mucin 1 gene has deletion, insertion, and substitution of amino acid(s), in addition to the variation of the number of tandem repeats, and thus it is highly variable. A part with variable numbers of tandem repeats in the center region is referred to as a VNTR region, and has many O type glycans. Meanwhile, N type glycans and O type glycans are present in a peptide region close to the membrane other than the tandem repeats in the center region.

The mucin 1 having a 3' sulfonated core 1 carbohydrate chain of the present invention is a novel compound, and is not limited, as long as it has the 3' sulfonated core 1 carbohydrate chain and the unique tandem repeat consisting of twenty amino acids (that is, PDTRPAPGSTAPPAHGVTSA). The 3' sulfonated core 1 carbohydrate chain of the mucin 1 having a 3' sulfonated core 1 carbohydrate chain is richly contained in the O type glycan of the VNTR region of the mucin 1 having a 3' sulfonated core 1 carbohydrate chain.

An example of an amino acid sequence of a mucin 1 protein containing an N-terminal region, a center region, a transmembrane region, and a C-terminal cytoplasmic region is exhibited in SEQ ID NO: 16. However, a mucin 1 is a variable, and thus the amino acid sequence of a mucin 1 having a 3' sulfonated core 1 carbohydrate chain is not limited to the amino acid sequence of SEQ ID NO: 16. Further, the number of the tandem repeats is not limited, but is preferably 1 to 200, more preferably 5 to 150, even more preferably 20 to 130, and most preferably 25 to 125. Further, in the amino acid sequence of the N-terminal region, the amino acid sequence of the repeat and the amino acid sequence other than the repeat in the center region, the amino acid sequence of the transmembrane region, and the amino acid sequence of the C-terminal cytoplasmic region in the mucin 1 having a 3' sulfonated core 1 carbohydrate chain, the mucin 1 having a 3' sulfonated core 1 carbohydrate chain of the present invention may contain mutation(s). For example, it may be an amino acid in which 1 to 100 amino acids of the amino acid sequence of SEQ ID NO: 16 are deleted, substituted, and/or added.

Meanwhile, if the mucin 1 having a 3' sulfonated core 1 carbohydrate chain of the present invention is subjected to a treatment with sialidase, the reactivity for lectin which binds to a 3' sulfonated core 1 carbohydrate chain as described below is increased. As sialic acid bound to the carbohydrate chain of a mucin 1 is removed, a sterically masked 3' sulfonated core 1 carbohydrate chain becomes exposed. This is believed to be how this phenomenon happens.

The molecular weight of the mucin 1 having a 3' sulfonated core 1 carbohydrate chain of the present invention is not limited, but it may be 20 kD to 1000 kD, for example. The mucin 1 having a 3' sulfonated core 1 carbohydrate chain may be a membrane-bound mucin 1 having a 3' sulfonated core 1 carbohydrate chain which is bound to a cell membrane, a secretory mucin 1 having a 3' sulfonated core 1 carbohydrate chain, or a partial peptide thereof. Therefore, body fluids of a breast cancer patient can contain the membrane-bound mucin 1 having a 3' sulfonated core 1 carbohydrate chain, the secretory mucin 1 having a 3' sulfonated core 1 carbohydrate chain, or the partial peptide thereof.

The cells or tissues, in which the mucin 1 having a 3' sulfonated core 1 carbohydrate chain of the present invention is expressed, are also not limited, but mammalian glands, lungs, uteruses, or pancreases can be mentioned, for example. In particular, as described in the Examples that are described below, the mucin 1 having a 3' sulfonated core 1 carbohydrate chain is expressed in breast cancer cells, breast cancer tissue, or cell lines derived from breast cancer.

The mucin 1 having a 3' sulfonated core 1 carbohydrate chain of the present invention may, for example, be isolated from specimens. In particular, it can be isolated from specimens derived from the living body (for example, blood such as serum or plasma, or breast cancer tissue) which contains the mucin 1 having a 3' sulfonated core 1 carbohydrate chain, or sub-cultured cells derived from breast cancer or culture supernatants thereof. Purification can be made from the specimens using ammonium sulfate precipitation, ion-exchange column chromatography, hydrophobic column chromatography, gel filtration column chromatography, affinity column chromatography, dialysis, lyophilization, or the like. In particular, it can be purified by using an affinity column prepared using an antibody binding to the 3' sulfonated core 1 carbohydrate chain or an antibody binding to the mucin 1 having a 3' sulfonated core 1 carbohydrate chain, and by performing affinity chromatography.

The mucin 1 having a 3' sulfonated core 1 carbohydrate chain of the present invention can be used as a standard substance in a method for analyzing the mucin 1 having a 3' sulfonated core 1 carbohydrate chain mentioned later. In addition, a kit for analyzing the mucin 1 having a 3' sulfonated core 1 carbohydrate chain may contain the mucin 1 having a 3' sulfonated core 1 carbohydrate chain as a standard substance.

In the specification, the 3' sulfonated core 1 carbohydrate chain more specifically means "SO₃⁻-3Galβ1 → 3GalNAcα1 → Ser (Thr)". Examples of the carbohydrate chain containing a 3' sulfonated core 1 carbohydrate chain, which is contained in a mucin 1, include, although are not limited thereto,

3' sulfonated core 1 carbohydrate chain represented by the formula (I),
[Chemical Formula 1]

SO₃⁻-3Ga*β*1→3GalNAcα1→Ser (Th r) (I)

(hereinbelow, referred to as "3' sulfonated core 1 carbohydrate chain (I)"),
3' sulfonated core 1 carbohydrate chain represented by the formula (II),
[Chemical Formula 2] 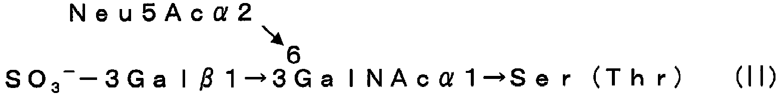 (hereinbelow, referred to as "3' sulfonated core 1 carbohydrate chain structure (II)"), and
3' sulfonated core 1 carbohydrate chain represented by the formula (III),
[Chemical Formula 3] 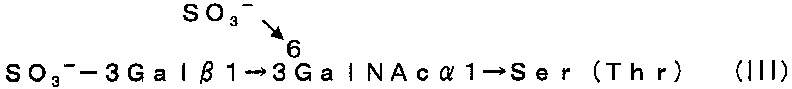 (hereinbelow, referred to as "3' sulfonated core 1 carbohydrate chain structure (III)"),

Meanwhile, in the present specification, the "core 1 carbohydrate chain" means "Galβ1 → 3GalNAcα1 → R".

A mucin 1 containing the aforementioned 3' sulfonated core 1 carbohydrate chain (I), 3' sulfonated core 1 carbohydrate chain (II), and 3' sulfonated core 1 carbohydrate chain (III) is hardly present in the blood of a healthy subject but is increased in the blood of a breast cancer patient.

Further, the amount of the 3' sulfonated core 1 carbohydrate chain (I) in the carbohydrate chain in the mucin 1 having a 3' sulfonated core 1 carbohydrate chain varies from breast cancer patient to breast cancer patient. It may be, although not particularly limited, 0.1 to 99% by mol, 1 to 50% by mol, or 5 to 15% by mol, for example. Further, the amount of 3' sulfonated core 1 carbohydrate chain (II) in the carbohydrate chain in the mucin 1 having a 3' sulfonated core 1 carbohydrate chain also varies from breast cancer patient to breast cancer patient. It may be, although not particularly limited, 0.1 to 99% by mol, 1 to 50% by mol, or 1 to 10% by mol, for example. Further, the amount of 3' sulfonated core 1 carbohydrate chain (III) in the carbohydrate chain in the mucin 1 having a sulfonated core 1 carbohydrate chain also varies from breast cancer patient to breast cancer patient. It may be, although not particularly limited, 0.1 to 99% by mol, 1 to 50% by mol, or 5 to 15% by mol, for example.

The mucin 1 having a 3' sulfonated core 1 carbohydrate chain of the present invention includes one or two or more mucin 1 having a 3' sulfonated core 1 carbohydrate chain that is selected from the group consisting of the 3' sulfonated core 1 carbohydrate chain (I), the 3' sulfonated core 1 carbohydrate chain (II), and the 3' sulfonated core 1 carbohydrate chain (III).

The mucin 1 having a 3' sulfonated core 1 carbohydrate chain of the present invention may have a carbohydrate chain other than the 3' sulfonated core 1 carbohydrate chain (I), the 3' sulfonated core 1 carbohydrate chain (II), and the 3' sulfonated core 1 carbohydrate chain (III), and examples thereof include "Neu5Acα2,3Galβ1,3GalNAcα carbohydrate chain", "Neu5Acα2 → 3Galβ1 → 3(Neu5Acα2 → 6)GalNAcβ → R", and "Neu5Acα2 → 3Galβ1 → 3(Neu5Acα2 → 3Galβ1 → 4GlcNAcβ1 → 6)GalNAcβ → R", "Neu5Acα2 → 8Neu5Acoα2 → 3Galβ1 → 4GlcNAcβ1 → 3Galβ1 → 3GalNAc → R" or "Neu5Acα2 → 8Neu5Acoα2 → 3Galβ1 → 3GlcNAcβ → R".

### [2] Method for analyzing a mucin 1 having a Sulfo-3Galβ1-3GalNAc-R carbohydrate chain

The first embodiment of the method for analyzing the mucin 1 having a 3' sulfonated core 1 carbohydrate chain of the present invention is characterized in that it has (A) a step of bringing a 3' sulfonated core 1 carbohydrate chain binding probe capable of binding to the Sulfo-3Galβ1-3GalNAc-R carbohydrate chain into contact with a sample to be tested (hereinbelow, it may be referred to as the contact step (A-a)), a step of bringing a 3' sulfonated core 1 carbohydrate chain mucin 1 peptide binding probe capable of binding to the mucin 1 having a Sulfo-3Galβ1-3GalNAc-R carbohydrate chain or a mucin 1 binding probe capable of binding to a mucin 1 into contact with the sample to be tested (hereinbelow, it may be referred to as the contact step (A-b)), and a step of detecting a conjugate of the mucin 1 having a Sulfo-3Galβ1-3GalNAc-R carbohydrate chain and the probe.

Further, the second embodiment of the method for analyzing the mucin 1 having a 3' sulfonated core 1 carbohydrate chain of the present invention is characterized in that it has (B) a step of bringing a 3' sulfonated core 1 carbohydrate chain mucin 1 peptide binding probe capable of binding to the mucin 1 having a Sulfo-3Galβ1-3GalNAc-R carbohydrate chain into contact with a sample to be tested (hereinbelow, it may be referred to as the contact step (B-a)), a step of bringing a 3' sulfonated core 1 carbohydrate chain mucin 1 peptide binding probe capable of binding to the mucin 1 having a Sulfo-3Galβ1-3GalNAc-R carbohydrate chain, a mucin 1 binding probe capable of binding to a mucin 1, or a mucin 1 carbohydrate chain binding probe capable of binding to a carbohydrate chain possessed by mucin 1 into contact with the sample to be tested (hereinbelow, it may be referred to as the contact step (B-b)), and a step of detecting a conjugate of the mucin 1 having a Sulfo-3Galβ1-3GalNAc-R carbohydrate chain and the probe.

Meanwhile, the "conjugate of the mucin 1 having a Sulfo-3Galβ1-3GalNAc-R carbohydrate chain and the probe" may be either a conjugate of the mucin 1 having a Sulfo-3Galβ1-3GalNAc-R carbohydrate chain and one probe or a conjugate of the mucin 1 having a Sulfo-3Galβ1-3GalNAc-R carbohydrate chain and two or more probes.

As for the 3' sulfonated core 1 carbohydrate chain binding probe used for the aforementioned contact step (A-a), a 3' sulfonated core 1 carbohydrate chain binding lectin or a 3' sulfonated core 1 carbohydrate chain binding antibody can be used. More specifically, as for the 3' sulfonated core 1 carbohydrate chain binding probe, a 3' sulfonated core 1 carbohydrate chain binding lectin or a lectin fragment having a carbohydrate chain binding region thereof, or a 3' sulfonated core 1 carbohydrate chain binding antibody or an antibody fragment having an antigen binding region thereof can be used.

As for the 3' sulfonated core 1 carbohydrate chain mucin 1 peptide binding probe that is used for the above contact step (A-b), a 3' sulfonated core 1 carbohydrate chain mucin 1 peptide binding antibody can be mentioned. Specifically, a 3' sulfonated core 1 carbohydrate chain mucin 1 peptide binding antibody or an antibody fragment having the antigen binding region thereof can be used. Further, as for the mucin 1 binding probe, a mucin 1 peptide binding antibody or a mucin 1 carbohydrate chain peptide binding antibody can be mentioned. Specifically, a mucin 1 peptide binding antibody or an antibody fragment having the antigen binding region thereof, or a mucin 1 carbohydrate chain peptide binding antibody or an antibody fragment having the antigen binding region thereof can be used.

As for the 3' sulfonated core 1 carbohydrate chain mucin 1 peptide binding probe that is used for the above contact step (B-a), a 3' sulfonated core 1 carbohydrate chain mucin 1 peptide binding antibody can be mentioned. Specifically, a 3' sulfonated core 1 carbohydrate chain mucin 1 peptide binding antibody or an antibody fragment having the antigen binding region thereof can be used.

As for the 3' sulfonated core 1 carbohydrate chain mucin 1 peptide binding probe that is used for the above contact step (B-b), a 3' sulfonated core 1 carbohydrate chain mucin 1 peptide binding antibody can be mentioned. Specifically, a 3' sulfonated core 1 carbohydrate chain mucin 1 peptide binding antibody or an antibody fragment having an antigen binding region thereof can be used. Further, as for the mucin 1 binding probe, a mucin 1 peptide binding antibody or a mucin 1 carbohydrate chain peptide binding antibody can be mentioned. Specifically, a mucin 1 peptide binding antibody or an antibody fragment having the antigen binding region thereof, or a mucin 1 carbohydrate chain peptide binding antibody or an antibody fragment having the antigen binding region thereof can be used. Further, as for the mucin 1 carbohydrate chain binding probe, a mucin 1 carbohydrate chain binding lectin or a mucin 1 carbohydrate chain binding antibody can be mentioned. Specifically, a mucin 1 carbohydrate chain binding lectin or a lectin fragment having the carbohydrate chain binding region thereof, or a mucin 1 carbohydrate chain binding antibody or an antibody fragment having the antigen binding region thereof can be used.

Hereinbelow, the 3' sulfonated core 1 carbohydrate chain binding probe, 3' sulfonated core 1 carbohydrate chain mucin 1 peptide binding probe, mucin 1 binding probe, and mucin 1 carbohydrate chain binding probe are explained in detail.

### «3' Sulfonated core 1 carbohydrate chain binding probe»

The 3' sulfonated core 1 carbohydrate chain binding probe is a probe for recognizing the 3' sulfonated core 1 carbohydrate chain. In other words, the 3' sulfonated core 1 carbohydrate chain binding probe is a probe which binds only to the 3' sulfonated core 1 carbohydrate chain as an epitope, and although the recognized epitope is overlapped with the 3' sulfonated core 1 carbohydrate chain mucin 1 peptide probe, which recognizes a combination of the 3' sulfonated core 1 carbohydrate chain and the peptide (amino acid) of mucin 1, they are not identical to each other. Further, the 3' sulfonated core 1 carbohydrate chain binding probe is not limited if it can recognize a carbohydrate chain containing "SO₃⁻-3Galβ1 → 3GalNAcα1 → Ser (Thr)". However, it is a probe sufficiently capable of recognizing one or more of the 3' sulfonated core 1 carbohydrate chain (I), the 3' sulfonated core 1 carbohydrate chain (II), or the 3' sulfonated core 1 carbohydrate chain (III) described above, for example.

### (3' Sulfonated core 1 carbohydrate chain binding lectin)

The 3' sulfonated core 1 carbohydrate chain binding lectin is not limited if it is a lectin capable of binding to a 3' sulfonated core 1 carbohydrate chain, and examples thereof include lectin having affinity for a Sulfo-3Galβ1-3GalNAc-R carbohydrate chain and lectin having affinity for a Sulfo-3Galβ1 carbohydrate chain. In other words, lectin having affinity for other carbohydrate chains can also be included in the 3' sulfonated core 1 carbohydrate chain binding lectin that is used in the present invention, as long as it is lectin capable of binding to a 3' sulfonated core 1 carbohydrate chain.

Examples of the lectin having affinity for a Sulfo-3Galβ1-3GalNAc-R carbohydrate chain include, although are not limited thereto, the galectin-1, the galectin-3, the galectin-4, the galectin-6, the galectin-8, and the galectin-9. The origin of the galectins is not limited either, and even a galectin originating from various animals can be also used. In those galectins, an amino acid sequence required for binding to a Sulfo-3Galβ1-3GalNAc-R carbohydrate chain is preserved, and they can bind to the 3' sulfonated core 1 carbohydrate chain of mucin 1. In particular, the galectin-4 is preferable. Because the galectin-4 has high binding specificity to the 3' sulfonated core 1 carbohydrate chain of mucin 1 (Non Patent Literature 4).

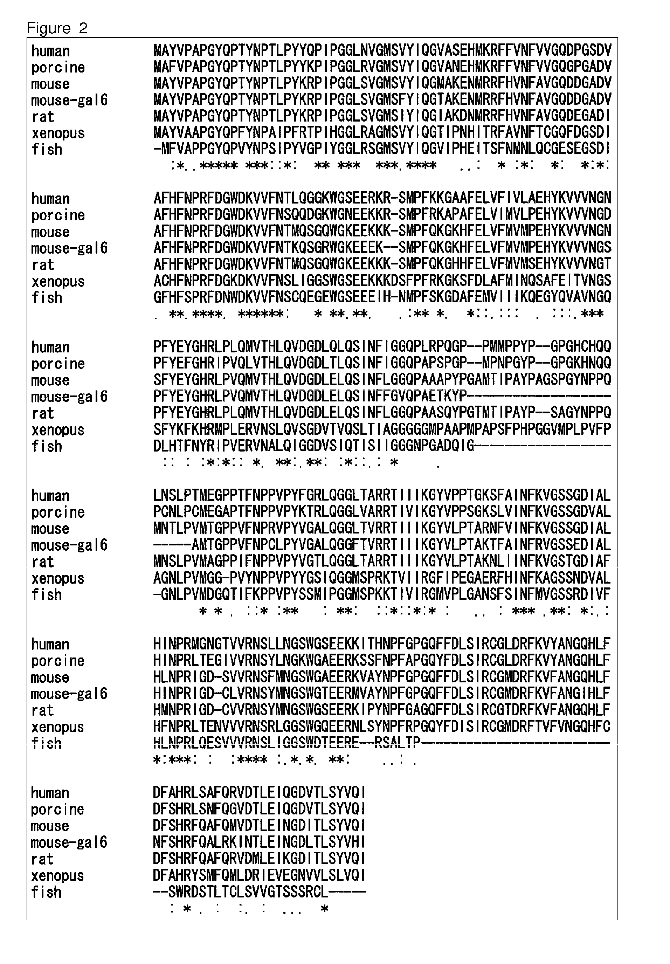

An origin of the galectin-4 is not limited as long as binding to a 3' sulfonated core 1 carbohydrate chain can be obtained. Examples thereof include the galectin-4 originating from mammals (for example, human, chimpanzee, pig tailed monkey, common marmoset, pig, cow, horse, goat, sheep, dog, cat, rabbit, panther, mouse, and rat), birds (for example, chicken), amphibians (for example, *Xenopus laevis),* reptiles (for example, green anole lizard), or fish (for example, salmon). As illustrated in Fig. 2, the galectin-4 of mammals such as human (SEQ ID NO: 2), pig (SEQ ID NO: 4), mouse (SEQ ID NO: 6), or rat (SEQ ID NO: 8) has a high amino acid sequence homology. Specifically, the homology rate between human and pig is 80%, the homology rate between human and rat is 77%, and the homology rate between human and mouse is 76%. Thus, it can be used without limitation as the 3' sulfonated core 1 carbohydrate chain binding probe for the analysis method of the present invention. Further, the homology rate between human and *Xenopus laevis* (SEQ ID NO: 10) is 55% and the homology rate between human and salmon (SEQ ID NO: 12) is 49%, and thus it can be used as the 3' sulfonated core 1 carbohydrate chain binding probe for the analysis method of the present invention.

Further, when compared to the amino acid sequence of the galectin-4, the galectin-6 (SEQ ID NO: 14) of a mouse illustrated in Fig. 2 has the homology rate of 70% with a human, as the linker portion connecting two carbohydrate binding sites is different. However, the amino acid sequence homology in two carbohydrate binding sites is high and the amino acid sequence required for binding to a Sulfo-3Galβ1-3GalNAc-R carbohydrate chain is preserved, and thus it can bind to a Sulfo-3Galβ1-3GalNAc-R carbohydrate chain.

Specific examples of the lectin capable of binding to the 3' sulfonated core 1 carbohydrate chain that can be used for the analysis method of the present invention include the following:
(1) A polypeptide selected from the group consisting of a polypeptide consisting of the amino acid sequence represented by the SEQ ID NO: 2, a polypeptide consisting of the amino acid sequence represented by the SEQ ID NO: 4, a polypeptide consisting of the amino acid sequence represented by the SEQ ID NO: 6, a polypeptide consisting of the amino acid sequence represented by the SEQ ID NO: 8, a polypeptide consisting of the amino acid sequence represented by the SEQ ID NO: 10, a polypeptide consisting of the amino acid sequence represented by the SEQ ID NO: 12, and a polypeptide consisting of the amino acid sequence represented by the SEQ ID NO: 14,
(2) A polypeptide capable of binding to a Sulfo-3Galβ1-3GalNAc-R carbohydrate chain, in which the polypeptide is selected from the group consisting of a polypeptide containing the amino acid sequence represented by the SEQ ID NO: 2, a polypeptide containing the amino acid sequence represented by the SEQ ID NO: 4, a polypeptide containing the amino acid sequence represented by the SEQ ID NO: 6, a polypeptide containing the amino acid sequence represented by the SEQ ID NO: 8, a polypeptide containing the amino acid sequence represented by the SEQ ID NO: 10, a polypeptide containing the amino acid sequence represented by the SEQ ID NO: 12, and a polypeptide containing the amino acid sequence represented by the SEQ ID NO: 14,
(3) A polypeptide capable of binding to a Sulfo-3Galβ1-3GalNAc-R carbohydrate chain, in which the polypeptide consists of an amino acid sequence having substitution, deletion, and/or insertion of 1 to 100 amino acids (preferably 1 to 50 amino acids, more preferably 1 to 20 amino acids, even more preferably 1 to 10 amino acids, and most preferably 1 to several amino acids) in the amino acid sequence selected from the group consisting of the amino acid sequence represented by the SEQ ID NO: 2, the amino acid sequence represented by the SEQ ID NO: 4, the amino acid sequence represented by the SEQ ID NO: 6, the amino acid sequence represented by the SEQ ID NO: 8, the amino acid sequence represented by the SEQ ID NO: 10, the amino acid sequence represented by the SEQ ID NO: 12, and the amino acid sequence represented by the SEQ ID NO: 14, or the polypeptide contains the amino acid sequence, and
(4) A polypeptide capable of binding to a Sulfo-3Galβ1-3GalNAc-R carbohydrate chain, in which the polypeptide consists of an amino acid sequence having the homology of 50% or higher (preferably amino acid sequence of 70% or higher, and preferably amino acid sequence of 80% or higher, and most preferably amino acid sequence of 90% or higher) with the amino acid sequence selected from the group consisting of the amino acid sequence represented by the SEQ ID NO: 2, the amino acid sequence represented by the SEQ ID NO: 4, the amino acid sequence represented by the SEQ ID NO: 6, the amino acid sequence represented by the SEQ ID NO: 8, the amino acid sequence represented by the SEQ ID NO: 10, the amino acid sequence represented by the SEQ ID NO: 12, and the amino acid sequence represented by the SEQ ID NO: 14, or the polypeptide contains the amino acid sequence.

Further, the 3' sulfonated core 1 carbohydrate chain binding lectin may be the one isolated and purified from cells or tissues, and also it may be the one produced by genetic engineering by using a recombinant. For example, the galectin-4 of the Examples explained below is obtained from E. coli by using a recombinant product, and it binds to a Sulfo-3Galβ1-3GalNAc-R carbohydrate chain. Further, a lectin fragment containing a domain, which is capable of binding to a Sulfo-3Galβ1-3GalNAc-R carbohydrate chain, can also be produced by genetic engineering.

As the 3' sulfonated core 1 carbohydrate chain binding lectin, mushroom lectin (Agaricus bisporus Agglutinin: ABA) can also be exemplified. ABA having a molecular weight of about 64 kDa is lectin originating from mushrooms, and it consists of four subunits with 143 amino acids (molecular weight of about 16 kDa). ABA binds not only to the 3' sulfonated core 1 carbohydrate chain but also to the "Galβ1 → 3GalNAcα1 → Ser (Thr)" (core 1 carbohydrate chain) and "Siaα2 → 3(6)Galβ1 → 3GalNAcα → Ser (Thr)", and it can be used as the 3' sulfonated core 1 carbohydrate chain binding lectin for the analysis method of the present invention.

### (3' Sulfonated core 1 carbohydrate chain binding antibody)

As for the 3' sulfonated core 1 carbohydrate chain binding antibody, it is not limited if it is an antibody capable of binding to the 3' sulfonated core 1 carbohydrate chain. Examples thereof include an antibody capable of binding to a Sulfo-3Galβ1-3GalNAc-R carbohydrate chain and an antibody capable of binding to a Sulfo-3Galβ1 carbohydrate chain. Since the antibody capable of binding to the 3' sulfonated core 1 carbohydrate chain can only bind to the 3' sulfonated core 1 carbohydrate chain, it can bind to a glycoprotein or a glycolipid having the 3' sulfonated core 1 carbohydrate chain.

The antibody capable of binding to the 3' sulfonated core 1 carbohydrate chain can be produced by a known method, except that a 3' sulfonated core 1 carbohydrate chain or a glycoprotein having the 3' sulfonated core 1 carbohydrate chain is used as an immuno antigen. For example, a monoclonal antibody can be produced via the method by Koehler and Milstein (Nature 256: 495-497, 1975). Further, a polyclonal antibody is an antigen in which a 3' sulfonated core 1 carbohydrate chain or a glycoprotein having the 3' sulfonated core 1 carbohydrate chain is present by itself or conjugated to BSA or KLH, and it is admixed with an adjuvant such as Freund's complete adjuvant and used for regular intradermal immunization for rabbits. At the point in time where there is an increased antibody titer in the blood, the blood is sampled and it can be used directly as an anti-serum or after purification of the antibody, according to a known method.

### «3' Sulfonated core 1 carbohydrate chain mucin 1 peptide binding probe»

The 3' sulfonated core 1 carbohydrate chain mucin 1 peptide binding probe is a probe which recognizes a combination of the 3' sulfonated core 1 carbohydrate chain and mucin 1 peptide (amino acid) as one epitope. In other words, the 3' sulfonated core 1 carbohydrate chain mucin 1 peptide binding probe is a probe capable of recognizing an epitope consisting of a combination of the 3' sulfonated core 1 carbohydrate chain and mucin 1 peptide, and although the epitope is overlapped with the 3' sulfonated core 1 carbohydrate chain mucin 1 peptide probe which recognizes the 3' sulfonated core 1 carbohydrate chain, they are not identical to each other. Further, the 3' sulfonated core 1 carbohydrate chain mucin 1 peptide binding probe is not limited if it can recognize a combination of the carbohydrate chain containing "SO₃⁻-3Galβ1 → 3GalNAcα1 → Ser (Thr)" and a peptide as one epitope. However, it is sufficiently a probe capable of recognizing, as one epitope, a combination of one or more of the 3' sulfonated core 1 carbohydrate chain (I), the 3' sulfonated core 1 carbohydrate chain (II), or the 3' sulfonated core 1 carbohydrate chain (III) described above and a peptide, for example.

### (3' Sulfonated core 1 carbohydrate chain mucin 1 peptide binding antibody)

The 3' sulfonated core 1 carbohydrate chain mucin 1 peptide binding antibody is an antibody which binds to the mucin 1 having a 3' sulfonated core 1 carbohydrate chain, does not bind to mucin 1 lacking a 3' sulfonated core 1 carbohydrate chain, and it does not bind to the 3' sulfonated core 1 carbohydrate chain only. In other words, it is an antibody capable of recognizing a combination of the 3' sulfonated core 1 carbohydrate chain and the peptide of mucin 1 as one epitope. As for the 3' sulfonated core 1 carbohydrate chain mucin 1 peptide binding antibody, the 1D1 monoclonal antibody manufactured by Sigma, which is used in the Examples described below, can be mentioned. The monoclonal antibody is obtained by using a partial, purified product of mucin 1 from pleural fluid of a human breast cancer patient as an immuno antigen, and it binds to an epitope consisting of a combination of the 3' sulfonated core 1 carbohydrate chain of mucin 1 and the peptide. Meanwhile, regarding the 3' sulfonated core 1 carbohydrate chain mucin 1 peptide binding antibody, there is an antibody which exhibits strong affinity for the 3' sulfonated core 1 carbohydrate chain and the peptide of mucin 1, and also exhibits cross-reactivity for a combination of a carbohydrate chain other than the 3' sulfonated core 1 carbohydrate chain and the peptide. Such antibody is also included in the 3' sulfonated core 1 carbohydrate chain mucin 1 peptide binding antibody.

Further, the 3' sulfonated core 1 carbohydrate chain mucin 1 peptide binding antibody can be produced by a known method, except that the mucin 1 having a 3' sulfonated core 1 carbohydrate chain is used as an immuno antigen. For example, a monoclonal antibody can be produced via the method by Koehler and Milstein (Nature 256: 495-497, 1975). In hybridoma screening, by selecting a hybridoma producing a monoclonal antibody which: binds to the mucin 1 having the 3' sulfonated core 1 carbohydrate chain, does not bind to mucin 1 not having the 3' sulfonated core 1 carbohydrate chain, and does not bind to the 3' sulfonated core 1 carbohydrate chain only, the antibody capable of binding to the mucin 1 having a 3' sulfonated core 1 carbohydrate chain is obtainable. Further, a polyclonal antibody is an antigen in which the mucin 1 having a 3' sulfonated core 1 carbohydrate chain is present by itself or conjugated to BSA or KLH, and it is admixed with an adjuvant such as Freund's complete adjuvant and used for regular intradermal immunization for rabbits. At the point in time where there is an increased antibody titer in the blood, the blood is sampled, and by adsorbing the antibody capable of binding to a mucin 1 lacking the 3' sulfonated core 1 carbohydrate chain and the antibody capable of binding to the 3' sulfonated core 1 carbohydrate chain by using an affinity column or the like, the polyclonal antibody capable of binding to the mucin 1 having a 3' sulfonated core 1 carbohydrate chain is obtainable.

### «Mucin 1 binding probe»

Although a mucin 1 binding probe is a probe capable of binding to a mucin 1, it is a probe having an epitope to be recognized that is substantially different from the 3' sulfonated core 1 carbohydrate chain binding probe or the 3' sulfonated core 1 carbohydrate chain mucin 1 peptide binding probe. In other words, the epitope of a mucin 1 binding probe is not substantially overlapped with the epitope of the 3' sulfonated core 1 carbohydrate chain binding probe or the 3' sulfonated core 1 carbohydrate chain mucin 1 peptide binding probe.

### (Mucin 1 peptide binding antibody or mucin 1 carbohydrate chain peptide binding antibody)

The mucin 1 binding probe is specifically a mucin 1 peptide binding antibody, or a mucin 1 carbohydrate chain peptide binding antibody, and it is not limited if it can bind to mucin 1 lacking a 3' sulfonated core 1 carbohydrate chain and the mucin 1 having a 3' sulfonated core 1 carbohydrate chain. However, examples thereof include an antibody which binds to mucin 1 lacking the 3' sulfonated core 1 carbohydrate chain and the mucin 1 having a 3' sulfonated core 1 carbohydrate chain, but does not bind to the 3' sulfonated core 1 carbohydrate chain only. In other words, it includes the mucin 1 peptide binding antibody which specifically recognizes the peptide of mucin 1 as an epitope and the mucin 1 carbohydrate chain peptide binding antibody which specifically recognizes a combination of the carbohydrate chain and peptide of mucin 1 as an epitope.

Regarding the mucin 1 peptide binding antibody or mucin 1 carbohydrate chain peptide binding antibody described above, except that the mucin 1 is used as an immuno antigen, it can be produced by a known method. For example, a monoclonal antibody can be produced via the method by Koehler and Milstein (Nature 256: 495-497, 1975). Further, a polyclonal antibody is an antigen in which mucin 1 is present by itself or conjugated to BSA or KLH, and it is admixed with an adjuvant such as Freund's complete adjuvant and used for regular intradermal immunization for rabbits. At the point in time where there is an increased antibody titer in the blood, the blood is sampled and it can be used directly as an anti-serum or after purification of the antibody, according to a known method.

Further, as for the mucin 1 peptide binding antibody or mucin 1 carbohydrate chain peptide binding antibody, various anti-mucin 1 antibodies have been obtained before and are commercially available, and for example, monoclonal antibody 115D8 or monoclonal antibody DF3 that are used for detection of CA15-3 can also be used.

Further, the monoclonal antibody KL-6 used for detection of KL-6 can also be used as a mucin 1 carbohydrate chain peptide binding antibody for the analysis method of the present invention. The KL-6 antibody has been reported as a monoclonal antibody capable of recognizing the amino acid sequence PDTRPAP and Neu5Acα2, 3Galβ1, 3GalNAcα carbohydrate chain (sialylated carbohydrate chain) of mucin 1 (Non Patent Literature 5). Inventors of the present invention found that the KL-6 antibody exhibits strong affinity for the Neu5Acα2-3(SO₃⁻-6)Galβ1-4GlcNAcβ1-3Galβ1-3GalNAc carbohydrate chain, the Neu5Acα2-3Galβ1-3(SO₃⁻-6)GalNAc carbohydrate chain, and the SO₃⁻-6[SO₃⁻-3Galβ1-3GalNAc] carbohydrate chain, as well as the sialylated carbohydrate chain. It also exhibits affinity for the 3' sulfonated core 1 carbohydrate chain (I) and the 3' sulfonated core 1 carbohydrate chain (II). In other words, the KL-6 antibody is an antibody exhibiting strong affinity for carbohydrate chains other than the 3' sulfonated core 1 carbohydrate chain and the peptide of mucin 1, but it is also an antibody exhibiting affinity for the 3' sulfonated core 1 carbohydrate chain and the peptide of mucin 1. Such an antibody is conveniently included in the mucin 1 carbohydrate chain peptide binding antibody for the analysis method of the present invention. However, it is also possible to use it as a 3' sulfonated core 1 carbohydrate chain mucin 1 peptide binding antibody.

### «Mucin 1 carbohydrate chain binding probe»

The mucin 1 carbohydrate chain binding probe is a probe which binds only to a carbohydrate chain contained in mucin 1, and it is a probe which binds only to a carbohydrate chain other than the 3' sulfonated core 1 carbohydrate chain. In other words, the epitope of the mucin 1 carbohydrate chain binding probe is not substantially overlapped with the epitope of the 3' sulfonated core 1 carbohydrate chain binding probe or 3' sulfonated core 1 carbohydrate chain mucin 1 peptide binding probe, but it may be overlapped with an epitope of the mucin 1 binding probe described above. Further, as it can also bind to the aforementioned glycoprotein having a carbohydrate chain other than mucin 1, it is not a probe specifically binding to a mucin 1.

### (Mucin 1 carbohydrate chain binding lectin)

The mucin 1 carbohydrate chain binding lectin is not limited as long as it is lectin having affinity for a carbohydrate chain other than the 3' sulfonated core 1 carbohydrate chain contained in mucin 1. Examples thereof include lectin capable of recognizing Neu5Acα2, 3Galβ1, 3GalNAcα carbohydrate chains contained in mucin 1. Specific examples thereof include Jacalin and mushroom lectin (ABA), and because ABA can also bind to the 3' sulfonated core 1 carbohydrate chain, it may also be used as the 3' sulfonated core 1 carbohydrate chain binding lectin, and it may also be used as the mucin 1 carbohydrate chain binding lectin. Further, examples of the mucin 1 carbohydrate chain binding lectin include lectin capable of recognizing Neu5Acα2 → 3Galβ1 → 3(Neu5Acα2 → 6)GalNAcβ → R or Neu5Acα2 → 3Galβ1 → 3(Neu5Acα2 → 3Galβ1 → 4GlcNAcβ1 → 6)GalNAcβ → R. Further examples include lectin capable of binding to "Neu5Acα2 → 8Neu5Acα2 → 3Gal (hereinbelow, it may also be referred to as α2,8 disialyl carbohydrate chain).

### (Mucin 1 carbohydrate chain binding antibody)

The mucin 1 carbohydrate chain binding antibody is not limited as long as it is an antibody capable of binding to a carbohydrate chain contained in mucin 1 other than the 3' sulfonated core 1 carbohydrate chain. Examples thereof include an antibody capable of recognizing the Neu5Acα2,3Galβ1,3GalNAcα carbohydrate chains, Neu5Acα2 → 3Galβ1 → 3(Neu5Acα2 → 6)GalNAcβ → R, or Neu5Acα2 → 3Galβ1 → 3(Neu5Acα2 → 3Galβ1 → 4GlcNAcβ1 → 6)GalNAcβ → R contained in a mucin 1. Further, examples thereof include an S2-566 monoclonal antibody and a 1E6 monoclonal antibody capable of binding to Neu5Acα2 → 8Neu5Acoα2 → 3Galβ1 → 4GlcNAcβ1 → 3Galβ1 → 3GalNAc → R, which is an α2,8 disialyl carbohydrate chain.

As for the probe used for the analysis method of the present invention, a lectin fragment having a carbohydrate chain binding region of the aforementioned lectin or the antibody fragment having an antigen binding region of the aforementioned antibody can be used.

As for the lectin fragment having a carbohydrate chain binding region of lectin, a polypeptide having a carbohydrate chain binding region can be produced by genetic engineering by using a recombinant, for example.

Further, examples of the antibody fragment having the antigen binding region of an antibody include F(ab')₂, Fab', Fab, and Fv. Those antibody fragments can be obtained by, for example, digesting an antibody with a proteinase (for example, pepsin and papain) according to a common method and purifying it by common method for protein separation and purification.

### «Sandwich method»

The first embodiment and the second embodiment of the analysis method of the present invention can be carried out as follows, although it is not limited thereto.

The first embodiment of the method for analyzing the mucin 1 having a 3' sulfonated core 1 carbohydrate chain of the present invention includes the contact step (A-a), the contact step (A-b), and the detection step. With regard to the contact step (A-a) and the contact step (A-b), either one of the contact step (A-a) and the contact step (A-b) can be carried out first. As such, in terms of the order of carrying out the contact step (A-a), the contact step (A-b), and the detection step, there are the following two embodiments.
(1) The contact step (A-a) is carried out, the contact step (A-b) is carried out, and then the detection step is carried out.
(2) The contact step (A-b) is carried out, the contact step (A-a) is carried out, and then the detection step is carried out.

Further, the second embodiment of the method for analyzing the mucin 1 having a 3' sulfonated core 1 carbohydrate chain of the present invention includes the following two embodiments.
(1) The contact step (B-a) is carried out, the contact step (B-b) is carried out, and then the detection step is carried out.
(2) The contact step (B-b) is carried out, the contact step (B-a) is carried out, and then the detection step is carried out.

When the first embodiment of the analysis method of the present invention is carried out according to the sandwich method, the following two embodiments can be mentioned. Meanwhile, the second embodiment can be carried out in a similar manner by substituting the probe.
(1) Sandwich method in which Step (a) for bringing a 3' sulfonated core 1 carbohydrate chain binding probe into contact with a sample to be tested; Step (b) for bringing a 3' sulfonated core 1 carbohydrate chain mucin 1 peptide binding probe or a mucin 1 binding probe into contact with the sample to be tested; and a step for detecting a conjugate of the mucin 1 having a 3' sulfonated core 1 carbohydrate chain and the probe are carried out in this order (hereinbelow, referred to as the Sandwich method (1)).
(2) Sandwich method in which Step (b) for bringing a 3' sulfonated core 1 carbohydrate chain mucin 1 peptide binding probe or a mucin 1 binding probe into contact with a sample to be tested; Step (a) for bringing a 3' sulfonated core 1 carbohydrate chain binding probe into contact with the sample to be tested; and a step for detecting a conjugate of the mucin 1 having a 3' sulfonated core 1 carbohydrate chain and the probe are carried out in this order (hereinbelow, referred to as the Sandwich method (2)).

For example, the sandwich method can be carried out as follows.

### (i) First reaction step

A capture probe (first probe) is immobilized to an insoluble carrier, such as a micro plate or beads. Next, the insoluble carrier is blocked with an appropriate blocking agent (for example, bovine serum albumin or gelatin) to prevent a non-specific adsorption to the capture probe or the insoluble carrier. Thereafter, the sample to be tested, which contains the mucin 1 having a 3' sulfonated core 1 carbohydrate chain and a first reaction buffer are added to an insoluble carrier (micro plate or beads) on which the capture probe (first probe) is immobilized, and the capture probe (first probe) is allowed to be brought into contact with the mucin 1 having a 3' sulfonated core 1 carbohydrate chain for binding. After that, the antigen not bound to the capture antibody or impurities are washed with a suitable washing liquid (for example, a phosphate buffer solution containing surfactant).

### (ii) Second reaction step

A label probe (second probe) labeled with an enzyme such as horse radish peroxidase (HRP) is added to a probe binding to a mucin 1 having a 3' sulfonated core 1 carbohydrate chain so as to bind the label probe to the captured mucin 1 having a 3' sulfonated core 1 carbohydrate chain. According to the reaction, a complex of the capture probe - the mucin 1 having a 3' sulfonated core 1 carbohydrate chain - the label probe is formed on the insoluble carrier (micro plate or beads).

Further, a "biotin labeled probe" conjugated with biotin or an "unlabel probe" may also be used as the second antibody instead of the above "label probe," which is labeled with an enzyme.

### (iii) Detection step

The insoluble carrier (micro plate or beads) is washed with a wash liquid and then a colorimetric substrate or a luminescent substrate for the enzyme of the labeled antibody is added. A signal is then detected after allowing the reaction between the enzyme and the substrate.

Alternatively, if the unlabeled probe is used instead of directly labeling the second antibody, it is also possible that an antibody capable of binding to the second probe is labeled and then a signal is detected. Further, if the biotin labeled antibody is used, an enzyme labeled avidin can be used so as to detect the signal.

In the case of the Sandwich method (1) of the first embodiment, as a capture probe (that is, the first probe), the 3' sulfonated core 1 carbohydrate chain binding lectin, the lectin fragment having the carbohydrate chain binding region thereof, a 3' sulfonated core 1 carbohydrate chain binding antibody or an antibody fragment having the antigen binding region thereof, or a mixture thereof is used. As a label probe (that is, second probe), a 3' sulfonated core 1 carbohydrate chain mucin 1 peptide binding antibody or an antibody fragment having the antigen binding region thereof, a mucin 1 peptide binding antibody or an antibody fragment having the antigen binding region thereof, a mucin 1 carbohydrate chain peptide binding antibody or an antibody fragment having the antigen binding region thereof, or a mixture thereof is used.

Further, in the case of the Sandwich method (2) of the first embodiment, as a capture probe (that is, the first probe), the 3' sulfonated core 1 carbohydrate chain mucin 1 peptide binding antibody or an antibody fragment having the antigen binding region thereof, a mucin 1 peptide binding antibody or an antibody fragment having the antigen binding region thereof, a mucin 1 carbohydrate chain peptide binding antibody or an antibody fragment having the antigen binding region thereof, or a mixture thereof is used, and as a label probe (that is, the second probe), the 3' sulfonated core 1 carbohydrate chain binding lectin or a lectin fragment having the carbohydrate chain binding region thereof, or the 3' sulfonated core 1 carbohydrate chain binding antibody or an antibody fragment having the antigen binding region thereof, or a mixture thereof is used.

In the case of the Sandwich method (1) of the second embodiment, as a capture probe (that is, the first probe), the 3' sulfonated core 1 carbohydrate chain mucin 1 peptide binding antibody or an antibody fragment having the antigen binding region thereof, or a mixture thereof is used, and as a label probe (that is, the second probe), the 3' sulfonated core 1 carbohydrate chain mucin 1 peptide binding antibody or an antibody fragment having the antigen binding region thereof, a mucin 1 peptide binding antibody or an antibody fragment having the antigen binding region thereof, a mucin 1 carbohydrate chain peptide binding antibody or an antibody fragment having the antigen binding region thereof, a mucin 1 carbohydrate chain binding lectin or a lectin fragment having the carbohydrate chain binding region thereof, a mucin 1 carbohydrate chain binding antibody or an antibody fragment having the antigen binding region thereof, or a mixture thereof is used.

Further, in the case of the Sandwich method (2) of the second embodiment, as a capture probe (that is, the first probe), the 3' sulfonated core 1 carbohydrate chain mucin 1 peptide binding antibody or an antibody fragment having the antigen binding region thereof, a mucin 1 peptide binding antibody or an antibody fragment having the antigen binding region thereof, a mucin 1 carbohydrate chain peptide binding antibody or an antibody fragment having the antigen binding region thereof, a mucin 1 carbohydrate chain binding lectin or a lectin fragment having the carbohydrate chain binding region thereof, a mucin 1 carbohydrate chain binding antibody or an antibody fragment having the antigen binding region thereof, or a mixture thereof is used, and as a label probe (that is, the second probe), the 3' sulfonated core 1 carbohydrate chain mucin 1 peptide binding antibody or an antibody fragment having the antigen binding region thereof, or a mixture thereof is used.

The sandwich method can be carried out by enzyme immunoassay, chemiluminescent immunoassay, electro chemiluminescent immunoassay or radioimmunoassay. Therefore, examples of the enzyme that labels the antibody include horse radish peroxidase (HRP), alkaline phosphatase, β-galactosidase, and luciferase. Furthermore, in addition to the enzyme, luminescent substances such as acridinium derivatives, fluorescent substances such as europium, electro chemiluminescent substances such as ruthenium complex, radioactive substances such as I¹²⁵, and the like may be used as a label substance. In addition, the substrate and the luminescent-inducing substance may be properly selected in accordance with the label substance. Furthermore, the labeled antibody, which may be used in the present invention, also includes an antibody which is conjugated with a substance such as hapten or a low molecular weight peptide as a detection marker, or lectin that may be used in the signal detection of the antigen antibody reaction.

The third embodiment of the method for analyzing the mucin 1 having a 3' sulfonated core 1 carbohydrate chain of the present invention includes the contact step (A-a) of the first embodiment but not the contact step (A-b) and the detection step. Specifically, there can be a method in which the mucin 1 having a 3' sulfonated core 1 carbohydrate chain is allowed to bind to the 3' sulfonated core 1 carbohydrate chain binding probe and then it is dissociated and recovered from the 3' sulfonated core 1 carbohydrate chain binding probe. When the 3' sulfonated core 1 carbohydrate chain binding lectin is used as a 3' sulfonated core 1 carbohydrate chain binding probe, a lectin affinity column is used. When a 3' sulfonated core 1 carbohydrate chain binding antibody is used as a 3' sulfonated core 1 carbohydrate chain binding probe, an antibody affinity column is used.

By binding the mucin 1 having a 3' sulfonated core 1 carbohydrate chain in a sample using the lectin affinity column or antibody affinity column followed by elution, the mucin 1 having a 3' sulfonated core 1 carbohydrate chain in a sample is recovered. The recovered mucin 1 having a 3' sulfonated core 1 carbohydrate chain can be detected by a general protein detecting method (for example, protein staining after gel electrophoresis, or protein detection by UV meter), or a detecting method specific for mucin 1 (for example, detection by an enzyme immunoassay using an anti-mucin 1 antibody).

The fourth embodiment of the method for analyzing the mucin 1 having a 3' sulfonated core 1 carbohydrate chain of the present invention includes the contact step (A-a) and the detection step but not the contact step (A-b).

Specifically, there can be a method in which step (A-a) for bringing the 3' sulfonated core 1 carbohydrate chain binding probe capable of binding to the mucin 1 having a 3' sulfonated core 1 carbohydrate chain into contact with a sample to be tested and a step for detecting a conjugate of the mucin 1 having a 3' sulfonated core 1 carbohydrate chain and the 3' sulfonated core 1 carbohydrate chain binding probe are carried out. As for the 3' sulfonated core 1 carbohydrate chain binding probe, a 3' sulfonated core 1 carbohydrate chain binding lectin or a 3' sulfonated core 1 carbohydrate chain binding antibody can be used.

The fourth embodiment can be carried out, for a case in which the 3' sulfonated core 1 carbohydrate chain binding antibody is used, by latex agglutination immunoassay, a fluorescent antibody method, radioimmunoassay, an immunoprecipitation method, an immunohistological staining method, or Western blot. For a case in which the 3' sulfonated core 1 carbohydrate chain binding lectin is used, it can be performed by using a lectin blot.

### (Samples to be tested)

Examples of the sample to be tested that is used in the analyzing method of the present invention include biological samples derived from a human body that possibly contains mucin 1 having a 3' sulfonated core 1 carbohydrate chain. Examples of the sample to be tested include: urine, blood, serum, plasma, spinal fluid, saliva, cells, tissue or organ, or preparations thereof (for example, a biopsy sample, particularly a biopsy sample from a breast cancer patient). The sample to be tested is preferably blood, serum, plasma, or a biopsy sample of a lacteal gland, particularly preferable is blood, serum, or plasma. Blood, serum, or plasma is appropriate as a sample to be tested for detecting breast cancer, because mucin 1 having a 3' sulfonated core 1 carbohydrate chain is released into the blood in breast cancer patients, whereas it is almost entirely not present in the blood, serum, or plasma of healthy subjects.

A liquid sample such as urine, blood, serum, plasma, spinal fluid, or saliva may be used after diluted with an appropriate buffer, depending on the analysis method. In addition, a solid sample such as cells, tissue or organs is dissolved with an appropriate buffer in the amount of about 2 to 10 times the volume of the solid sample, and a suspension or a supernatant thereof may be used in the analysis method as it is, or after further dilution.

The "sample derived from the human body possibly containing mucin 1 having a 3' sulfonated core 1 carbohydrate chain" as used herein includes a sample to be tested containing mucin 1 and a sample to be tested possibly containing mucin 1. This is because a sample to be tested that is derived from a patient having breast cancer or possibly suffering from breast cancer is sometimes used for the analysis.

### [3] Method for detecting or monitoring breast cancer

According to the method for analyzing mucin 1 having a 3' sulfonated core 1 carbohydrate chain, it is possible to detect or monitor breast cancer.

### (Method for detecting breast cancer)

It is possible to detect or diagnose whether or not a subject (patient) has breast cancer, by measuring an amount of the mucin 1 having a 3' sulfonated core 1 carbohydrate chain in a sample from the subject (patient) using the method for analyzing mucin 1 having a 3' sulfonated core 1 carbohydrate chain, and comparing the amount to the amount of mucin 1 having a 3' sulfonated core 1 carbohydrate chain found in a healthy subject.

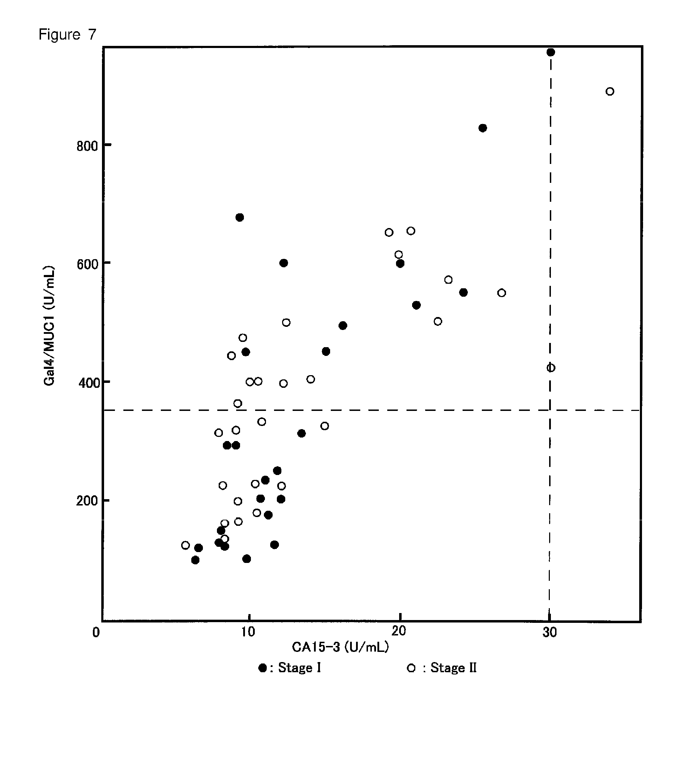

Further, as illustrated in Table 2 and Fig. 7, the method for detecting breast cancer of the present invention exhibited high detection rates of 40% and 55% for primary breast cancer patients at stage I and stage II, compared to the detection rate obtained by the measurement of CA15-3 of a related art.

### (Method for monitoring breast cancer)

Further, according to the method for analyzing mucin 1 having a 3' sulfonated core 1 carbohydrate chain, by measuring an amount of the mucin having a 3' sulfonated core 1 carbohydrate chain in a sample of a breast cancer patient undergoing treatments, monitoring of breast cancer can be performed (for example, for monitoring the malignancy of the breast cancer, the degree of progress of the breast cancer, the metastasis of the breast cancer, and recurrence of the breast cancer).

As listed in Table 1, a very high positive ratio is exhibited in a patient with metastatic breast cancer compared to a positive ratio obtained by measurement of CA15-3 of a related art. Thus, the method for analyzing mucin 1 having a 3' sulfonated core 1 carbohydrate chain of the present invention can be used as a method for predicting or monitoring metastasis of breast cancer.

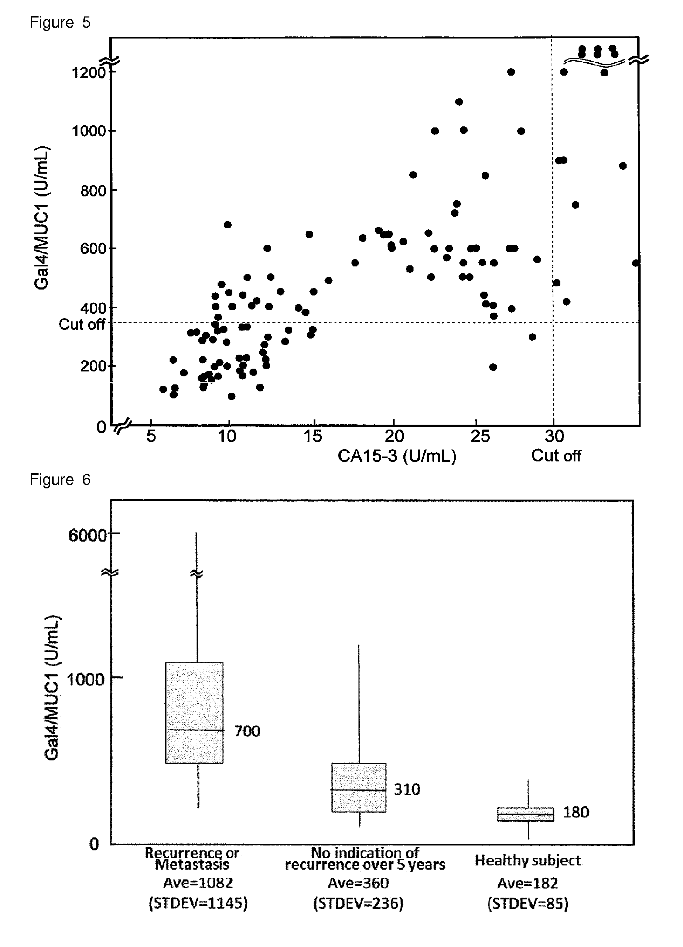

Further, as illustrated in Fig. 6, a patient having metastasis or recurrence has a higher measurement value of the mucin 1 having a 3' sulfonated core 1 carbohydrate chain compared to a patient with no indication of recurrence over 5 years or longer. Thus, the method for analyzing mucin 1 having a 3' sulfonated core 1 carbohydrate chain of the present invention can be used as a method for monitoring recurrence or metastasis of breast cancer.

### [4] Kit for analyzing mucin 1 having a Sulfo-3Galβ1-3GalNAc-R carbohydrate chain

A kit for analyzing mucin 1 having a 3' sulfonated core 1 carbohydrate chain of the present invention is a kit used for the method for analyzing mucin 1 having a 3' sulfonated core 1 carbohydrate chain. The kit for analyzing mucin 1 having a 3' sulfonated core 1 carbohydrate chain of the present invention can be used as a kit for detecting or monitoring breast cancer.

The first embodiment of the kit for analyzing the mucin 1 having a 3' sulfonated core 1 carbohydrate chain of the present invention includes a 3' sulfonated core 1 carbohydrate chain binding probe capable of binding to a Sulfo-3Galβ1-3GalNAc-R carbohydrate chain; and a 3' sulfonated core 1 carbohydrate chain mucin 1 peptide binding probe capable of binding to the mucin 1 having a Sulfo-3Galβ1-3GalNAc-R carbohydrate chain or a mucin 1 binding probe capable of binding to a mucin 1.

The second embodiment of the kit for analyzing the mucin 1 having a 3' sulfonated core 1 carbohydrate chain includes a 3' sulfonated core 1 carbohydrate chain mucin 1 peptide binding probe capable of binding to the mucin 1 having a Sulfo-3Galβ1-3GalNAc-R carbohydrate chain; and a mucin 1 binding probe capable of binding to a mucin 1 or a mucin 1 carbohydrate chain binding probe capable of binding to a carbohydrate chain possessed by mucin 1.

The third embodiment of the kit for analyzing the mucin 1 having a 3' sulfonated core 1 carbohydrate chain includes a 3' sulfonated core 1 carbohydrate chain binding probe capable of binding to a Sulfo-3Galβ1-3GalNAc-R carbohydrate chain.

As for the 3' sulfonated core 1 carbohydrate chain binding probe, a 3' sulfonated core 1 carbohydrate chain binding lectin or a lectin fragment having the carbohydrate chain binding region thereof, or a 3' sulfonated core 1 carbohydrate chain binding antibody or an antibody fragment having the antigen binding region thereof can be used. As for the 3' sulfonated core 1 carbohydrate chain mucin 1 peptide binding probe, a 3' sulfonated core 1 carbohydrate chain mucin 1 peptide binding antibody or an antibody fragment having the antigen binding region thereof can be used. As for the mucin 1 binding probe, mucin 1 peptide binding antibody or an antibody fragment having the antigen binding region thereof, or a mucin 1 carbohydrate chain peptide binding antibody or an antibody fragment having the antigen binding region thereof can be used. As for the mucin 1 carbohydrate chain binding probe, a mucin 1 carbohydrate chain binding lectin or a lectin fragment having the carbohydrate chain binding region thereof, or a mucin 1 carbohydrate chain binding antibody or an antibody fragment having the antigen binding region thereof can be used.

The probe may be linked to a carrier or may be dissolved in buffer solution, depending on the measurement method of an analysis kit. Examples of the carrier include sepharose, cellulose, agarose, dextran, polyacrylate, polystyrene, polyacrylamide, polymethacrylamide, a copolymer of styrene and divinylbenzene, polyamide, polyester, polycarbonate, polyethylene oxide, hydroxypropyl methylcellulose, polyvinyl chloride, polymethylacrylate, a copolymer of polystyrene and polystyrene, polyvinyl alcohol, polyacrylic acid, collagen, calcium alginate, latex, polysulfone, silica, zirconia, alumina, titania and ceramics. The shape of the carrier is not also particularly limited, and a carrier in the form of particulate beads, plate, gel and the like can be used.

For example, when the analysis method, corresponding to an immunological technique using a labeled antibody, is used (for example, enzyme immunoassay, chemiluminescent immunoassay, fluorescent antibody method, electro chemiluminescent immunoassay, and radioimmunoassay), the probe may be contained in the form of a labeled antibody or a labeled antibody fragment labeled with a labeling substance. Specific examples of the labeling substance include enzymes such as peroxidase, alkaline phosphatase, β-D-galactosidase or glucose oxidase, fluorescent substances such as fluorescein isothiocyanate or rare-earth metal chelates, radioactive isotopes such as ³H, ¹⁴C or ¹²⁵I, and further, biotin, avidin, and chemiluminescent substances. In the case of enzymes, chemiluminescent substances, or the like, they preferably contain an appropriately selected substrate and the like since they cannot generate a measurable signal on their own.

The analysis kit of the present invention may contain the mucin 1 having a 3' sulfonated core 1 carbohydrate chain as a standard substance. MUC1 mucin used as a standard substance can be purified by a common method which uses a culture supernatant of breast cancer cell lines such as YMB-1, a body fluid such as pleural fluid or abdominal fluid of a breast cancer patient as a raw material. Further, the kit of the present invention may contain a manual that describes use for the detection or monitoring of breast cancer. In addition, the descriptions for the use for detection or monitoring of breast cancer may be attached to the container of the analysis kit.

### Examples

The present invention will now be further illustrated by the following Examples, but they do not limit the scope of the present invention.

### <<Analysis example 1>>

In this analysis example, a 3' sulfonated core 1 carbohydrate chain in mucin 1 secreted from breast cancer cells (YMB-1) was analyzed. The carbohydrate chains in mucin 1 secreted from breast cancer cells (YMB-1) were released by β-elimination, and then were reduced and labeled by NaB³H₄. The obtained carbohydrate chains were fractionated by high-voltage paper electrophoresis at pH 5.4. Each resulting fraction was analyzed by Bio-Gel P-4 column chromatography, various lectin column chromatographies, and/or exo-glycosidase digestion including sialidase, so as to determine a carbohydrate chain structure contained in each fraction.

As a result, it was found that, as a carbohydrate chain containing a 3' sulfonated core 1 carbohydrate chain, the 3' sulfonated core 1 carbohydrate chain (I) represented by the following formula (I) was contained at about 7% by mol,
[Chemical Formula 4]

**SO₃⁻-3Gal*β*1→3GalNAc*α*1→Ser(Thr)** (I)

the 3' sulfonated core 1 carbohydrate chain (II) represented by the following formula (II) was contained at about 3% by mol
[Chemical Formula 5] 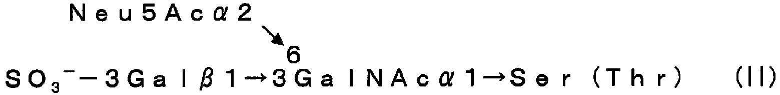 and the 3' sulfonated core 1 carbohydrate chain (III) represented by the following formula (III) was contained at about 10% by mol.
[Chemical Formula 6]

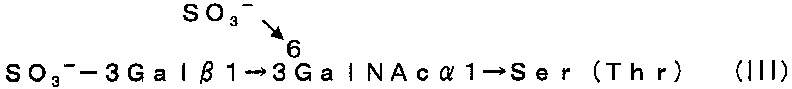

### «Example 1: Isolation of mucin 1 having a 3' sulfonated core 1 carbohydrate chain»

In this Example, mucin 1 having a 3' sulfonated core 1 carbohydrate chain was isolated and purified from a breast cancer cell line.

Cell culture liquid of the breast cancer cell line YMB-1 was collected, and after removing floating cells by centrifugation, it was concentrated and recovered by using a membrane with 100 kDa cut-off. The recovered culture liquid was applied to an anti-mucin 1 antibody affinity column equilibrated with PBS, washed with the same buffer solution, and eluted with an elution buffer solution (10 mM KH₂PO₄, 3 M NaCl, pH 2.5) to obtain a mucin 1 solution.

Meanwhile, the anti-mucin 1 affinity column was prepared by using CNBr-Sepharose (manufactured GEhealthcare), in accordance with the protocol recommended by the manufacturer.

### «Example 2: Preparation of galectin-4 and preparation of galectin-4-HRP»

In this Example, a recombinant human galectin-4 capable of binding to a 3' sulfonated core 1 carbohydrate chain was prepared.

First, cDNA encoding ORF of the galectin-4 was obtained by PCR from a human T-84 colonic epithelial cell cDNA library. The primers used include a sense primer: 5'-gctgtcgacATGGCCTATGTCCCCGCA-3' (SEQ ID NO: 17) and an antisense primer: 5'-cctaagcttTCTGGACATAGGACAAGG-3' (SEQ ID NO: 18). The obtained PCR fragment was linked to the SalI and HindIII sites of pQE-9 vector to obtain G4-pQE-9 vector. The E. coli M15 cell line was transformed with G4-pQE-9 vector. The transformed E. coli was cultured and an expression of the galectin-4 was induced with IPTG. The obtained E. coli was treated with lysozyme and a supernatant was obtained by centrifugation. From the obtained supernatant, the galectin-4 was purified by using a Ni-NTA resin and an asialofetuin-bound resin. Concentration of the purified galectin-4 was measured by a Bio-Rad Protein Assay and used for the following experiments.

### (Preparation of galectin-4-HRP)

The purified galectin-4 was labeled with horse radish peroxidase (HRP). For labeling with HRP, Peroxidase Labeling Kit-NH2 (Dojindo Laboratories) was used and the galectin-4-HRP was obtained according to the attached protocols.

### «Example 3: Construction of system for measuring mucin 1 having a 3' sulfonated core 1 carbohydrate chain»

In this Example, an immunological analysis method for mucin 1 having a 3' sulfonated core 1 carbohydrate chain based on a sandwich method was constructed.

50 µL of the anti-mucin monoclonal antibody (Sigma) diluted to a concentration of 4 µg/mL with 50 mM carbonate buffer solution (pH 9.6) was added to 96 well ELISA plates (Corning Incorporated) and was immobilized at 4°C for 16 hours. The surface was blocked with PBS containing 20% blocking reagent N102 (NOF CORPORATION) at 25°C for 1.5 hours. As a standard substance, the solution of YMB-1 mucin 1 having a 3' sulfonated core 1 carbohydrate chain obtained in Example 1 was diluted between 10 and 6000 times with a diluent in which 10% Carbofree blocking solution (Vector Laboratories) had been added to PBS-0.1% Tween-20 (PBS-T). Then, 50 µL was added and incubated at 25°C for 1.5 hours. After washing with PBS-T three times, 50 µL of the galectin-4-HRP diluted by a factor of 2000 was added and incubated at 25°C for 1.5 hours. Each well was washed with PBS-T four times, a 100 µL of TMB substrate solution (Pias Corporation) was added and incubated at 25°C for 15 minutes. After that, the reaction was terminated by using 2 N sulfuric acid solution, and the absorbance at 450 nm was measured by using a Plate CHAMELEON V (HIDEX Oy).

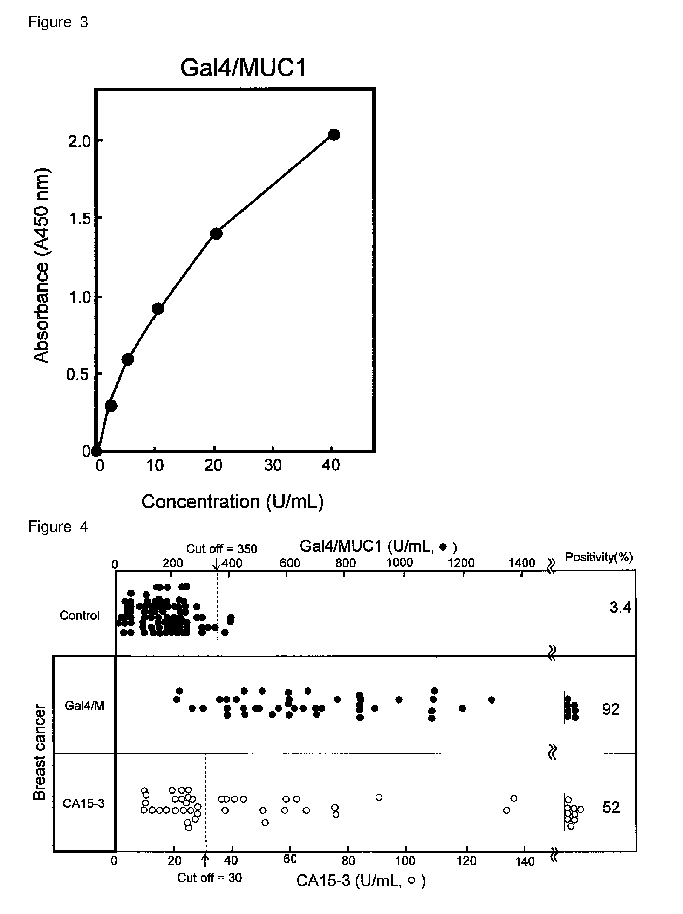

The obtained standard line is illustrated in Fig. 3. Meanwhile, the amount of mucin 1 having a 3' sulfonated core 1 carbohydrate chain was obtained by measuring the YMB-1 mucin 1 solution according to the "EITEST KL-6" (Eisai Co., Ltd.) and the calculation was made based on the obtained unit number.

### «Example 4: Measurement of serum from breast cancer patient»

For 48 test samples from patients with recurrent or metastatic breast cancer and 85 test samples from healthy subjects, a measurement of the mucin 1 having a 3' sulfonated core 1 carbohydrate chain was performed.

Except that 48 test samples from breast cancer patients or 85 test samples from healthy subjects are used instead of the YMB-1 mucin 1 solution, the procedures of Example 3 were repeated. From the standard line obtained from Example 3, the unit number in each test sample was calculated. The results are illustrated in Fig. 4. An average value of mucin 1 in serum of the healthy subject was 182 ± 85 U/mL. Meanwhile, an average value of mucin 1 in serum of the breast cancer patient was 1082 ± 1145 U/mL. As a result of having a cut-off value of 350 U/mL, the positive ratio was 92% in the breast cancer patients.

### «Comparative Example 1: Measurement of CA15-3 »

In this Comparative Example, CA15-3, which is a marker for breast cancer, was measured for 48 test samples of serum from breast cancer patients which have been measured in Example 4.

The measurement of CA15-3 was carried out using the "E test TOSOH II (CA15-3)" (TOSOH Corporation) in accordance with the protocol attached thereto.

Results are illustrated in Fig. 4. The positive ratio was 52% for 48 test samples of serum from the breast cancer patients.

The CA15-3 measurement value and the measurement value obtained by the analysis method of the present invention as measured in Example 4 were plotted (Fig. 5). It was recognized that many test samples found to be negative by CA15-3 can be determined as positive by the analysis method of the present invention.

### «Example 5: Prediction of recurrence in breast cancer patient»

In this Example, 24 test samples from breast cancer patients with no indication of recurrence over 5 years or longer after surgery were measured with the measurement method of Example 4, in addition to the 48 test samples from patients with recurrent or metastatic breast cancer and 85 test samples from healthy subjects which have been measured in Example 4. The results are illustrated in Fig. 6.

The breast cancer patients with no indication of recurrence over 5 years or longer after surgery definitely exhibited a lower measurement value than that of the patients with recurrent or metastatic breast cancer, and thus the method for analyzing the mucin 1 having a 3' sulfonated core 1 carbohydrate chain of the present invention is considered to be a useful marker for recurrence or metastasis of breast cancer.

### «Example 6»

In this Example, the mucin 1 having a 3' sulfonated core 1 carbohydrate chain in serum of eight patients with recurrent or metastatic breast cancer was measured according to the procedures of Example 4. The relation between an area of metastatic breast cancer and a positive ratio of Gal4/MUC1 for 56 test samples, that is, 48 test samples measured in Example 4 and 8 test samples measured in this Example, is listed in Table 1.

### «Comparative Example 2»

In this Comparative Example, CA15-3 in serum of eight patients with recurrent or metastatic breast cancer was measured according to the procedures of Comparative Example 1. The relation between an area of metastatic breast cancer and a positive ratio of CA15-3 for 56 test samples, that is, 48 test samples measured in Comparative Examples 4 and 8 test samples measured in this Comparative Example, is listed in Table 1.

**[Table 1]**

| Metastasis | Gal4/MUC1 | | CA15-3 | |
|---|---|---|---|---|
| Lung | 5/6 | (83 %) | 1/6 | (17 %) |
| Bone | 3/3 | (100 %) | 1/3 | (33 %) |
| Liver | 4/4 | (100 %) | 3/4 | (75 %) |
| Axillary lymph node | 8/8 | (100 %) | 3/8 | (38 %) |
| Local lymph node | 5/6 | (83 %) | 4/6 | (67 %) |
| Multiple metastasis | 16/17 | (94 %) | 10/17 | (59 %) |
| Chest lining | 2/2 | (100 %) | 2/2 | (100 %) |
| Thoracic vertebrate | 1/1 | (100 %) | 0/1 | (0 %) |
| Parasternal lymph node | 1/2 | (100 %) | 0/2 | (0 %) |
| Sacrum | 0/1 | (0 %) | 0/1 | (0 %) |
| skin | 1/1 | (100 %) | 0/1 | (0 %) |
| Brain | 1/1 | (100 %) | 0/1 | (0 %) |
| Recurrence (both breast) | 4/4 | (100 %) | 3/4 | (75 %) |
| Cut-off value | >350 | | >30 | |
| Positive ratio(n=56) | 51/56 | 91 % | 27/56 | 48 % |

The mucin 1 having a 3' sulfonated core 1 carbohydrate chain was positive at 91% (51/56) in the patients with recurrent or metastatic breast cancer. Further, the positive ratio was high regardless of an area of metastasis. Meanwhile, CA15-3 was positive at 48% (27/56) in the patients with recurrent or metastatic breast cancer, and thus the positive ratio was not high.

### «Example 7»

In this Example, the mucin 1 having a 3' sulfonated core 1 carbohydrate chain in serum of 54 patients with primary breast cancer was determined.

Except for the serum from 54 patients with primary breast cancer being used, the procedures of Example 4 were repeated. The positive ratio of the mucin 1 having a 3' sulfonated core 1 carbohydrate chain in the patient with primary breast cancer at stage I and stage II is listed in Table 2. When the cut-off value is set at 350 units, the positive ratio of the mucin 1 having a 3' sulfonated core 1 carbohydrate chain was 40% for stage I and 55% for stage II. Those positive ratios are very high compared to the positive ratio of CA15-3 described below.

### «Comparative Example 3»

In this Comparative Example, CA15-3 in serum of 54 patients with primary breast cancer was determined.

Except for the serum from 54 patients with primary breast cancer being used, the procedures of Comparative Example 1 were repeated. The positive ratio of CA15-3 in the patient with primary breast cancer at stage I and stage II is listed in Table 2. The positive ratio of CA15-3 was 4% for stage I and 10% for stage II.

**[Table 2]**

| Stage | Gal4/MUC1 | | CA15-3 | |
|---|---|---|---|---|
| I | 10/25 | (40 %) | 1/25 | (4 %) |
| II | 16/29 | (55 %) | 2/29 | (10 %) |
| Cut-off value | >350 | | >30 | |
| Positive ratio (n=54) | 26/54 | 48 % | 4/54 | 7 % |

Furthermore, the relation between the measurement values of the mucin 1 having a 3' sulfonated core 1 carbohydrate chain measured in Example 7 and the measurement values of CA15-3 measured in this Comparative Example was plotted in Fig. 7. It was found that, even in the sample that was negative according to CA15-3, there are many samples determined to be positive in terms of the mucin 1 having a 3' sulfonated core 1 carbohydrate chain.

### Industrial Applicability

The method for analyzing mucin 1 having a 3' sulfonated core 1 carbohydrate chain of the present invention can be used for the detection or monitoring of breast cancer. The analysis method or analysis kit of the present invention can be used as a marker for recurrence or metastasis of breast cancer.

The present invention is explained according to specific embodiments hereinabove. However, modifications or improvements that are obvious to a skilled person in the pertinent art are within the scope of the present invention.

## Claims

1. A method for analyzing a mucin 1 having a Sulfo-3Galβ1-3GalNAc-R carbohydrate chain, the method comprising:
(A) a step of bringing a 3' sulfonated core 1 carbohydrate chain binding probe binding to a Sulfo-3Galβ1-3GalNAc-R carbohydrate chain into contact with a sample to be tested,
a step of bringing a 3' sulfonated core 1 carbohydrate chain mucin 1 peptide binding probe binding to a mucin 1 having a Sulfo-3Galβ1-3GalNAc-R carbohydrate chain or a mucin 1 binding probe binding to a mucin 1 into contact with the sample to be tested, and
a step of detecting a conjugate of the mucin 1 having a Sulfo-3Galβ1-3GalNAc-R carbohydrate chain and the probe; or
(B) a step of bringing a 3' sulfonated core 1 carbohydrate chain mucin 1 peptide binding probe binding to a mucin 1 having a Sulfo-3Galβ1-3GalNAc-R carbohydrate chain into contact with a sample to be tested,
a step of bringing a 3' sulfonated core 1 carbohydrate chain mucin 1 peptide binding probe binding to the mucin 1 having a Sulfo-3Galβ1-3GalNAc-R carbohydrate chain, a mucin 1 binding probe binding to a mucin 1, or a mucin 1 carbohydrate chain binding probe binding to a carbohydrate chain possessed by mucin 1 into contact with the sample to be tested, and
a step of detecting a conjugate of the mucin 1 having a Sulfo-3Galβ1-3GalNAc-R carbohydrate chain and the probe.

2. A method for analyzing a mucin 1 having a Sulfo-3Galβ1-3GalNAc-R carbohydrate chain, the method comprising a step of bringing a 3' sulfonated core 1 carbohydrate chain binding probe binding to a Sulfo-3Galβ1-3GalNAc-R carbohydrate chain into contact with a sample to be tested.

3. The method for analyzing a mucin 1 having a Sulfo-3Galβ1-3GalNAc-R carbohydrate chain according to claim 2, the method comprising:
a step of bringing a 3' sulfonated core 1 carbohydrate chain binding probe binding to a Sulfo-3Galβ1-3GalNAc-R carbohydrate chain into contact with a sample to be tested; and
a step of detecting a conjugate of the mucin 1 having a Sulfo-3Galβ1-3GalNAc-R carbohydrate chain and the probe.

4. The method for analyzing a mucin 1 having a Sulfo-3Galβ1-3GalNAc-R carbohydrate chain according to any one of claims 1 to 3, wherein the 3' sulfonated core 1 carbohydrate chain binding probe is a 3' sulfonated core 1 carbohydrate chain binding lectin or a lectin fragment having a carbohydrate chain binding region thereof, or a 3' sulfonated core 1 carbohydrate chain binding antibody or an antibody fragment having an antigen binding region thereof, the 3' sulfonated core 1 carbohydrate chain mucin 1 peptide binding probe is a 3' sulfonated core 1 carbohydrate chain mucin 1 peptide binding antibody or an antibody fragment having an antigen binding region thereof, the mucin 1 binding probe is a mucin 1 peptide binding antibody or an antibody fragment having an antigen binding region thereof, or a mucin 1 carbohydrate chain peptide binding antibody or an antibody fragment having an antigen binding region thereof, and the mucin 1 carbohydrate chain binding probe is a mucin 1 carbohydrate chain binding lectin or a lectin fragment having a carbohydrate chain binding region thereof, or a mucin 1 carbohydrate chain binding antibody or an antibody fragment having an antigen binding region thereof.

5. The method for analyzing a mucin 1 having a Sulfo-3Galβ1-3GalNAc-R carbohydrate chain according to claim 4, wherein the 3' sulfonated core 1 carbohydrate chain binding lectin is selected from the group consisting of galectin-1, galectin-3, galectin-4, galectin-6, galectin-8, and galectin-9.

6. The method for analyzing a mucin 1 having a Sulfo-3Galβ1-3GalNAc-R carbohydrate chain according to claim 5, wherein the galectin-4 is galectin-4 originated from mammals, birds, amphibians, reptiles, or fishes.

7. A method for detecting or monitoring breast cancer, the method comprising the analysis of an amount of the mucin 1 having a Sulfo-3Galβ1-3GalNAc-R carbohydrate chain according to the analysis method described in any one of claims 1 to 6.

8. A kit for analyzing a mucin 1 having a Sulfo-3Galβ1-3GalNAc-R carbohydrate chain, the kit comprising a 3' sulfonated core 1 carbohydrate chain binding probe binding to a Sulfo-3Galβ1-3GalNAc-R carbohydrate chain.

9. A kit for analyzing a mucin 1 having a Sulfo-3Galβ1-3GalNAc-R carbohydrate chain, the kit comprising:
(A) a 3' sulfonated core 1 carbohydrate chain binding probe binding to a Sulfo-3Galβ1-3GalNAc-R carbohydrate chain; and a 3' sulfonated core 1 carbohydrate chain mucin 1 peptide binding probe binding to a mucin 1 having a Sulfo-3Galβ1-3GalNAc-R carbohydrate chain or a mucin 1 binding probe binding to a mucin 1, or
(B) a 3' sulfonated core 1 carbohydrate chain mucin 1 peptide binding probe binding to a mucin 1 having a Sulfo-3Galβ1-3GalNAc-R carbohydrate chain; and
a mucin 1 binding probe binding to a mucin 1, or a mucin 1 carbohydrate chain binding probe binding to a carbohydrate chain possessed by mucin 1.

10. The kit for analyzing a mucin 1 having a Sulfo-3Galβ1-3GalNAc-R according to claim 8 or 9, wherein the 3' sulfonated core 1 carbohydrate chain binding probe is a 3' sulfonated core 1 carbohydrate chain binding lectin or a lectin fragment having a carbohydrate chain binding region thereof, or a 3' sulfonated core 1 carbohydrate chain binding antibody or an antibody fragment having an antigen binding region thereof, the 3' sulfonated core 1 carbohydrate chain mucin 1 peptide binding probe is a 3' sulfonated core 1 carbohydrate chain mucin 1 peptide binding antibody or an antibody fragment having an antigen binding region thereof, the mucin 1 binding probe is a mucin 1 peptide binding antibody or an antibody fragment having an antigen binding region thereof, or a mucin 1 carbohydrate chain peptide binding antibody or an antibody fragment having an antigen binding region thereof, and the mucin 1 carbohydrate chain binding probe is a mucin 1 carbohydrate chain binding lectin or a lectin fragment having a carbohydrate chain binding region thereof, or a mucin 1 carbohydrate chain binding antibody or an antibody fragment having an antigen binding region thereof.

11. The kit for analyzing a mucin 1 having a Sulfo-3Galβ1-3GalNAc-R according to claim 10, wherein the 3' sulfonated core 1 carbohydrate chain binding lectin is selected from the group consisting of galectin-1, galectin-3, galectin-4, galectin-6, galectin-8, and galectin-9.

12. The kit for analyzing a mucin 1 having a Sulfo-3Galβ1-3GalNAc-R according to claim 11, wherein the galectin-4 is galectin-4 originated from mammals, birds, amphibians, reptiles, or fishes.

13. The kit for analyzing a mucin 1 having a Sulfo-3Galβ1-3GalNAc-R carbohydrate chain according to any one of claims 8 to 12, the kit further comprising a mucin 1 having a Sulfo-3Galβ1-3GalNAc-R carbohydrate chain.

14. A kit for detecting or monitoring breast cancer using the kit for analyzing a mucin 1 having a Sulfo-3Galβ1-3GalNAc-R carbohydrate chain described in any one of claims 8 to 13.

15. A mucin 1 having a Sulfo-3Galβ1-3GalNAc-R carbohydrate chain.
